# FASCICULE DE BREVET EUROPEEN

(11) **EP 1 115 434 B1**
(45) Date de publication et mention de la délivrance du brevet: **25.05.2005**
(21) Numéro de dépôt: 00958569.6
(22) Date de dépôt: 20.07.2000
(51) Int. Cl.: A61L 24/04, A61L 15/42

(54) **MOUSSE PROTEIQUE ADHESIVE A USAGE CHIRURGICAL ET/OU THERAPEUTIQUE**
EIWEISSSCHAUM KLEBSTOFF ZUR CHIRURGISCHEN UND/ODER THERAPEUTISCHEN VERWENDUNG
ADHESIVE PROTEIN FOAM FOR SURGICAL AND/OR THERAPEUTIC USES

(30) Priorité: 21.07.1999 FR 9909467; 21.07.1999 FR 9909461
(43) Date de publication de la demande: 18.07.2001
(73) Titulaire: Imedex Biomateriaux, 01600 Trevoux (FR)
(72) Inventeur: TAYOT, Jean-Louis, F-69890 La Tour de Salvagny (FR); BAYON, Yves, F-69100 Villeurbanne (FR); GRAVAGNA, Philippe, F-69540 Irigny (FR); DUBOIS, Michel, Marie, F-69110 Ste Foy lès Lyon (FR)
(74) Mandataire: Bernasconi, Jean Raymond
(86) Numéro de dépôt international: PCT/FR2000/002088
(87) Numéro de publication internationale: WO 2001/005443

(56) Documents cités:
- EP-A- 0 747 420
- WO-A-98/02098
- CH-A- 674 804
- FR-A- 2 754 268
- US-A- 2 584 082
- US-A- 4 442 655
- US-A- 4 612 332

## Description

La présente invention se situe dans le domaine des adhésifs biologiques, biodégradables et non toxiques destinés à un usage chirurgical et/ou thérapeutique.

D'une manière plus précise, la présente invention est relative à une mousse protéïque adhésive fluide biocompatible, biorésorbable et non toxique, à usage chirurgical et/ou thérapeutique.

Elle est également relative à une telle mousse renfermant des substances bioactives libérables en un site déterminé.

L'invention concerne par ailleurs un procédé pour l'obtention d'une telle mousse adhésive, ainsi qu'un kit pour sa préparation.

Elle concerne encore l'utilisation de la mousse adhésive en chirurgie et/ou à des fins thérapeutiques, notamment pour la protection de plaies et la liaison de tissus biologiques entre eux ou à un biomatériau implanté.

On connaît des colles biologiques pouvant adhérer aux tissus ou les fixer entre eux, en quelques minutes, sans utiliser des agrafes ou des sutures. Ces colles s'éliminent, en général après la cicatrisation de la plaie, par biodégradation, résorption ou par simple détachement sous forme de croûtes.

Différentes technologies ont été développées pour la formulation d'adhésifs tissulaires. Certains d'entre eux sont d'origine synthétique comme les colles à base de cyanoacrylates (2-butyl-cyanoacrylate, 2-octylcyanoacrylate) ou de polymères synthétiques et d'autres contiennent des matériaux biologiques comme le collagène ou la fibrine.

D'une manière générale, les adhésifs synthétiques sont utilisés pour l'étanchement de vaisseaux ou des poumons ainsi que pour « coller » les bords d'incisions cutanées. Les dérivés biologiques adhésifs tels que le collagène et la fibrine possèdent en outre des propriétés hémostatiques et agissent également en contrôlant les saignements.

Les colles cyanoacrylates se dégradent en formant des produits toxiques, même si les colles récemment développées sont moins nocives.

Elles conduisent à des produits cassant après polymérisation sur le site d'application. Elles restent en place 7 à 10 jours et sont éliminées par simple détachement, après cicatrisation. Leur temps de polymérisation est peu modulable, inférieur à 1 minute, et ne permet pas une utilisation souple de ces colles. Elles peuvent aussi facilement couler et, par suite, coller des tissus adjacents au site souhaité.

FOCAL (US 5,844,016) a décrit des adhésifs synthétiques reposant sur la polymérisation photochimique d'hydrogel de polyéthylène glycol (PEG). Leur procédé d'utilisation n'est pas pratique. En effet, ils impliquent une application en plusieurs étapes, sur le site opératoire, de la solution contenant l'initiateur photochimique (Eosine Y), de la solution de monomère (dérivé de PEG et d'acrylate) pouvant renfermer une substance biologiquement active, puis l'irradiation avec de la lumière jusqu'à obtenir un gel solide transparent et adhérent, après 40 à 60 secondes. Ce type d'adhésif nécessite ainsi l'application de plusieurs solutions qui, en raison de leur fluidité, peuvent facilement se répandre sur des sites adjacents au site cible.

Ces adhésifs ont aussi été décrits pour la délivrance ciblée de substances biologiquement actives (Vascular endothelial growth factor [VEGF], endothelial cell growth factor [ECGF], basic fibroblast growth factor [bFGF], bone morphogenic protein [BMP] ...) contenues dans leur réseau microparticulaire (FOCAL US 5 879 713).

BARD (WO 97/42986) a décrit un adhésif similaire à celui de FOCAL précité, pour lequel la polymérisation est induite par les u.v.

COHESION TECHNOLOGIES (US 5,874,500 ; US 5,744,545 ; US 5,550,187) a également décrit des colles liquides à base de PEG activé (ex. PEG comportant des groupes succinimidyles et maléimidyles) qui polymérisent après une simple application sur le site cible d'application, en un temps variable. Ces colles sont potentiellement toxiques et présentent l'inconvénient d'être fluides empêchant une application précise sur le site d'intervention.

CRYOLIFE a développé un autre type d'adhésif, à base d'un mélange d'albumine bovine et de glutaraldéhyde. Outre les effets toxiques connus de cet agent réticulant et du caractère antigénique de l'albumine bovine, cet adhésif présente également les problèmes de fluidité précités.

Les colles de fibrine, mélange de fibrinogène concentré et de thrombine, créent une matrice de fibrine qui est lentement dégradée par le système fibrinolytique endogène. Avant polymérisation, elles sont très fluides et, peuvent facilement couler, même si leur temps de réaction est ajustable en jouant sur la quantité totale de thrombine. Elles peuvent libérer des substances biologiques actives (ex. Zarge et coll., J. Surg. Res., 1997, 67, 4-8 ; Greisler et coll., Surgery, 1992, 112, 244-255 ; Gray et coll., Surg. Forum, 1993, 44, 394-396 ; Clinica, 1999, 848, 18).

Des dispositifs associant les colles de fibrine à des liposomes ont aussi été décrits (US 5,651,982).

Les colles de fibrines peuvent être vaporisées sur le site d'application, à l'aide d'un spray, et former un film de coagulum écumeux (US 5,607,694 ; WO 97/33646).

Des dispositifs complexes associant une protéine polymère de synthèse à un agent de réticulation ont été proposés comme adhésifs biologiques (US 5,817,303).

Enfin, plusieurs adhésifs à base de collagène ou de gélatine ont été décrits dans la littérature. Très tôt, la gélatine a été associée au résorcinol et au formaldéhyde ou au glutaraldéhyde pour conduire à un adhésif présentant également des propriétés hémostatiques (Tatooles et coll., Surgery, 1966, 60, 857-861 ; Braunwald et coll., Surgery, 1966, 59, 1024-1030 ; Guilmet et coll., J. Thorac. Cardiovasc. Surg., 1979, 77, 516-521). Avec ce type d'adhésif, il y a néanmoins un risque de relargage de formaldéhyde ou de glutaraldéhyde à l'origine de réactions toxiques, entraînant des nécroses tissulaires, ou des réactions moins sévères, conduisant à une mauvaise cicatrisation ou à son ralentissement.

Dans certaines formulations, le collagène est étroitement associé à de la thrombine (CoStasis de Cohesion technol. et Flo-Seal de Fusion).

Pour des applications en chirurgie, il peut être également modifié chimiquement avec des agents d'acylation ou de sulfonation pour que le collagène, ainsi transformé, puisse polymériser sur le site d'application, en présence ou non d'un initiateur (US 5,874,537 ; WO 97/42986).

Un adhésif obtenu à partir de collagène chauffé et, comme agent de réticulation, un polyaldéhyde macromoléculaire biodégradable a également été décrit (FR 2,754,267 ; FR 2,754,268).

Les colles à usage chirurgical et/ou thérapeutique, décrites dans la littérature, se présentent essentiellement sous forme liquide.

Un matériau lyophilisé non injectable, comprenant les éléments de la colle de fibrine (thrombine et fibrinogène) a été décrit (US 4 442 655). Un gaz inerte est éventuellement introduit dans la solution aqueuse réactive fibrinogène / thrombine pour alléger le matériau qui a un rôle hémostatique ou de support pour la délivrance de substances cicatrisantes et est principalement destiné au nettoyage des plaies. Un autre matériau lyophilisé non injectable comprenant entre autres les éléments de la colle de fibrine et du collagène a également été décrit dans la littérature, comme un hémostatique efficace et un adhésif (Nishida et coll., Geka Shinryo [Surgical Diagnosis Treatment], 1994, 36, 1449-1459 ; Ochiai et coll., Sanpujinka no Jissai [Obstetric and Gynecologic Practice], 1995, 44, 253-262 ; Schelling et coll., Ann. Surg., 1987, 205, 432-435 ; Shimamura et coll., The Clinical Report, 1994, 28, 2994-2507).

Certains adhésifs ont également été proposés sous forme de spray pour permettre une application plus homogène et plus discrète sur une surface importante. Cependant, l'utilisation de spray présente des inconvénients dont :
i) l'apport de quantités non négligeables de dioxyde de carbone ou d'autres gaz, entraînant des risques de surpression dangereuse et pouvant se révéler toxique pour des applications en chirurgie non invasive,
ii) le déplacement important du mélange adhésif sur le site de dépôt par le gaz propulseur de l'applicateur,
iii) le développement d'un applicateur spécial pour spray, augmentant sensiblement le prix de revient du dispositif adhésif et pouvant nécessiter un environnement plus complexe, notamment à cause de la connexion du dispositif à une source de gaz propulseur.

On connaît par ailleurs des mousses protéiques rigides obtenues par introduction d'un gaz (air) dans une solution de protéines puis séchage de la masse mousseuse à haute température, pour des panneaux de mousse isolants thermiques (US 2 584 082).

Une mousse, résultant de l'agitation d'une solution de protéines en présence d'air ou autre gaz inerte a également été incorporée dans des crèmes cosmétiques (CH 674 804).

On connaît encore des mousses de polysaccharides obtenues par mélange sous cisaillement après introduction d'un gaz dans la solution de polysaccharides, applicables par pulvérisation pour la cicatrisation de plaies ou comme barrière anti-adhérences post-opératoires (EP 747 420).

Aucune propriété adhésive sur des plaies ou des organes n'a été décrite pour ces mousses.

L'invention a pour objectif de fournir un adhésif ne présentant pas les inconvénients majeurs évoqués précédemment, en particulier, risques de toxicité, difficultés d'application notamment dues à la fluidité, à une application en plusieurs étapes et au temps de réactivité des composants, emploi de gaz propulseurs (spray), etc...

L'invention a ainsi pour objectif de fournir un adhésif qui soit fluide et éventuellement injectable, biocompatible, biorésorbable et non toxique, adapté à un usage chirurgical et / ou thérapeutique, stable dans le temps et pouvant être conservé dans des conditions relativement simples.

L'invention a aussi pour objectif de fournir un tel adhésif pour la liaison de tissus biologiques, y compris des tissus vivants, entre eux ou avec un biomatériau implanté ou encore pour le comblement de cavités tissulaires ou la protection de plaies tissulaires.

L'invention a également pour objectif de fournir un tel adhésif sous forme prête à l'emploi, d'utilisation simple et pratique, notamment injectable à l'aide de cathéters ou canules.

Un autre objectif de l'invention est de fournir un adhésif dont la structure facilite la colonisation tissulaire.

Un autre objectif est de fournir un adhésif dont la biodégradabilité est contrôlable dans le temps, après application.

Un autre objectif de l'invention est de fournir un adhésif pouvant contenir des substances biologiquement actives.

La présente invention a par ailleurs pour objectif de procurer un procédé pour la préparation d'un tel adhésif, qui soit facile à mettre en oeuvre et sans danger pour l'organisme receveur.

L'invention a en outre pour objectif de fournir des kits permettant une préparation simple et rapide d'un tel adhésif.

Ces objectifs ainsi que d'autres qui ressortiront de la description donnée ci-après, sont atteints à l'aide d'une mousse protéïque adhésive fluide biocompatible, biorésorbable et non toxique, à usage chirurgical et/ou thérapeutique, notamment pour la liaison de tissus biologiques entre eux ou à un biomatériau implanté et la protection / cicatrisation de plaies tissulaires, comprenant une matrice adhésive protéïque fluide biocompatible, biorésorbable et non toxique renfermant un gaz ou un mélange de gaz biocompatible et non toxique.

L'invention a pour objet un procédé pour la préparation d'une mousse adhésive telle que précitée, caractérisé en ce qu'il comprend le fait de mélanger extemporanément, de manière homogène:
- un composé protéique polymérisable/réticulable et potentiellement adhésif contenu dans une première seringue,
   à
- un agent de polymérisation/réticulation contenu dans une deuxième seringue,
   pour former un matériau fluide de matrice protéique adhésive biocompatible, biorésorbable et non toxique, et
- un gaz ou un mélange de gaz biocompatibles et non toxiques choisi parmi l'air, l'azote, l'oxygène ou le gaz carbonique ou le mélange d'un ou plusieurs de ces gaz, contenu dans la première et/ou la deuxième seringue et/ou une troisième seringue, avec ce matériau fluide de matrice protéique adhésive, ou avec un des constituants de base d'un tel matériau solubilisé en milieu aqueux,
   **par transfert en va-et-vient du mélange entre deux seringues.**

L'invention a aussi pour objet un kit pour la préparation d'une telle mousse adhésive, caractérisé en ce qu'il comprend:
- un composé protéique polymérisable/réticulable, potentiellement adhésif, solubilisé en milieu aqueux, dans une première seringue,
- un agent de polymérisation/réticulation dans une deuxième seringue,
   pour former une matrice protéique adhésive fluide biocompatible, biorésorbable et non toxique, et
- un gaz ou un mélange de gaz biocompatibles et non toxiques, choisi parmi l'air, l'azote, l'oxygène ou le gaz carbonique ou le mélange d'un ou plusieurs de ces gaz dans la première, deuxième et/ou dans une troisième seringue.

Les inventeurs ont mis en évidence, de manière surprenante, que l'on pouvait préparer des mousses fluides et adhésives, en incorporant extemporanément un gaz ou un mélange de gaz dans des 'colles' biologiques, pour obtenir des mousses protéiques prêtes à l'emploi, applicables notamment par injection, en utilisant différents dispositifs, tels que des canules ou des cathéters.

Les inventeurs ont mis en évidence, de manière tout à fait surprenante, la possibilité d'obtenir une mousse protéique adhésive biocompatible, biorésorbable et non toxique, fluide et injectable, adaptée à un usage chirurgical et/ou thérapeutique, à partir d'un composé protéique soit sous forme solubilisée en milieu aqueux, soit sous forme solide, notamment lyophilisé ou séché par un solvant volatil.

Les inventeurs ont montré, de manière tout à fait inattendue, que de telles mousses présentent des propriétés adhésives, notamment sur des tissus biologiques, y compris des tissus vivants, comparables aux 'colles' biologiques sous forme liquide, tout en étant plus élastiques et sont parfaitement tolérées par l'organisme receveur. Elles peuvent conserver leurs propriétés adhésives jusqu'à leur complète dégradation.

Ils ont également découvert que de telles mousses présentent des caractéristiques imprévues de colonisation rapide et efficace par des cellules de l'organisme receveur.

Ils ont également découvert, de manière tout autant imprévue, que de telles mousses adhésives pouvaient être appliquées avec une grande précision sur des tissus biologiques, pour leur liaison entre eux ou à un biomatériau implanté présentant des fonctions réactives vis-à-vis de la matrice adhésive, sans connaître les problèmes de coulure, habituellement rencontrés avec les colles biologiques liquides ou les risques de dispersion des colles par les gaz propulseurs des spray. Les dépôts de ces mousses adhésives sur les tissus sont en outre plus faciles à visualiser grâce à leur texture microporeuse particulière et à leur opacité, caractères se différenciant très sensiblement des colles liquides habituelles et des tissus humains ou animaux.

Ils ont également montré que certaines formulations de ces mousses perdent leur caractère 'collant' sur leur surface externe, après polymérisation des agents adhésifs, permettant une application sélective et précise de ces mousses sur les tissus cibles sans coller des tissus non désirés.

Ils ont également découvert que l'on peut facilement incorporer à ces mousses adhésives des substances biologiquement actives, éventuellement associées à un véhicule les protégeant au moins partiellement de modifications chimiques, potentiellement causées par les agents de polymérisation.

La présente invention va être décrite plus en détail ci-après.

Selon l'invention, par « matrice protéique adhésive », on entend un réseau formé d'un ou plusieurs composants protéïques présentant des propriétés adhésives et qui sont non toxiques, biocompatibles et biodégradables, ledit réseau renfermant un gaz ou un mélange de gaz biocompatibles et non toxiques.

Les propriétés adhésives de la matrice sont généralement acquises par un processus de polymérisation et/ou de réticulation de son ou ses constituants de base, de préférence, initié par un ou plusieurs agent(s) de polymérisation/réticulation fourni(s) avant la formation de la mousse.

Par « non toxique », on entend tout produit dont la toxicité est suffisamment faible pour permettre une utilisation en chirurgie et/ou en thérapeutique du corps humain ou animal, quel que soit le site d'application, en satisfaisant aux critères et normes imposés par la législation.

Par « biodégradable », on entend tout composant susceptible de disparaître par dégradation progressive (métabolisation).

La matrice adhésive peut correspondre, du point de vue de sa composition chimique, aux adhésifs et colles biologiques connus.

Elle peut ainsi consister ou comprendre un composé protéique (constituant de base), au moins partiellement polymérisé/réticulé qui est non toxique, biocompatible et biodégradable et qui possède des propriétés adhésives.

Le terme « composé protéique » désigne une protéine ou un mélange de protéines, éventuellement chimiquement modifiées, notamment par méthylation ou succinylation.

Le procédé selon l'invention permet l'obtention de matrices adhésives obtenues à partir d'une composition comprenant, d'une part, un composé protéique (constituant de base) polymérisable/réticulable, potentiellement adhésif et, d'autre part, un agent de polymérisation/réticulation, par leur mélange extemporané avant utilisation.

Conformément à l'invention, par « composé protéique polymérisable/réticulable potentiellement adhésif », on entend tout composé protéique tel que défini précédemment capable de développer, en présence d'eau, des propriétés adhésives par polymérisation et/ou réticulation sous l'effet d'un agent de polymérisation/réticulation.

Selon l'invention, l'agent de polymérisation/réticulation peut comprendre un composé ou un mélange de composés compatible avec le composé protéique polymérisable/réticulable pour provoquer la polymérisation/réticulation de celui-ci par un mélange extemporané, généralement en quelques minutes.

Le composé protéique est mis en oeuvre soit sous forme solubilisée en milieu aqueux, soit sous forme solide notamment de poudre ou de fibres.

L'agent de polymérisation/réticulation peut également être mis en oeuvre sous forme solubilisée en milieu aqueux ou sous forme pulvérulente, de préférence lyophilisée.

Les protéines mises en oeuvre aux fins de l'invention sont choisies de préférence parmi le collagène, la gélatine, l'albumine, l'élastine et te fibrinogène, et plus préférentiellement parmi le collagène et l'albumine. Le collagène est tout particulièrement préféré.

Le collagène utilisé aux fins de l'invention peut être indifféremment d'origine humaine ou animale, ou obtenu par des moyens de recombinaison génétique. Il peut s'agir de collagène de type I, III, IV ou V, ou encore de leur mélange en toute proportion.

Il peut s'agir de collagène natif, c'est-à-dire qui a conservé sa structure hélicoïdale d'origine, éventuellement chimiquement modifié par méthylation, par succinylation ou toute autre méthode connue, notamment pour le rendre plus soluble à pH physiologique, ou encore traité pour éliminer les télopeptides, notamment par digestion à la pepsine.

On peut utiliser également du collagène constitué majoritairement de chaînes α dont le poids moléculaire est voisin de 100 kDa, non hydrolysé. Dans ce cas, la structure hélicoïdale du collagène est dénaturée, au moins partiellement, par exemple par un chauffage modéré, en présence d'eau, notamment à une température comprise entre 40 et 70°C, dans des conditions douces de manière à éviter la dégradation par coupure hydrolytique de la gélatine ainsi formée, généralement moins de 10% des chaînes collagéniques ayant un poids moléculaire inférieur à 100 kDa.

Une telle gélatine est appelée ci-après « collagène chauffé », pour la distinguer de la gélatine du commerce qui peut également être utilisée aux fins de l'invention mais de manière non préférée.

Le collagène natif ou le collagène chauffé décrits précédemment est mis en oeuvre soit sous forme de fibres ou poudre sèche soit sous forme de solution aqueuse à une concentration comprise entre 1 et 5%, de préférence entre 2,5 et 4% en poids pour le collagène natif, entre 4 et 20% de préférence entre 5 et 16% en poids pour le collagène chauffé.

Le pH des solutions de collagène natif ou de collagène chauffé est de préférence neutre, plus préférentiellement compris entre 6 et 8.

Lorsque la matrice adhésive est obtenue à partir d'albumine, on l'utilise de préférence sous forme de poudre sèche soit sous forme d'une solution aqueuse à une concentration comprise entre 20 et 50% en poids, de préférence 40 à 50 %.

Dans le cas du fibrinogène, on utilise de préférence une poudre ou une solution aqueuse à une concentration comprise entre 10 et 20 %.

Conformément à la présente invention, l'agent de réticulation peut être choisi parmi des polymères réactifs naturels ou synthétiques, de préférence de poids moléculaire supérieur à 1000, tels que des polyaldéhydes macromoléculaires, des polymères hydrophiles, dont la diffusion ultérieure à partir de la colle est gênée par le poids moléculaire important, empêchant une toxicité directe immédiate.

Par « polymères réactifs », on entend des polymères capables de réagir avec les composés protéiques tels que définis précédemment, en particulier vis-à-vis de fonctions amine ou sulfhydryle qu'ils peuvent contenir.

Les polyaldéhydes macromoléculaires qui peuvent être mis en oeuvre selon l'invention comprennent des polyaldéhydes biodégradables d'origine naturelle, c'est-à-dire tout composé présentant plusieurs fonctions aldéhydiques dérivées d'un polymère naturel biodégradable.

Les polyaldéhydes peuvent être utilisés seuls ou en mélange, le terme « polyaldéhyde » utilisé ici désignant indifféremment un composé seul ou un mélange de plusieurs de ces composés.

Ces polyaldéhydes macromoléculaires peuvent être préparés par oxydation de polysaccharides ou de mucopolysaccharides notamment avec de l'acide périodique ou l'un de ses sels selon un procédé connu en soi.

Parmi les polysaccharides ou mucopolysaccharides convenant à la réalisation de l'invention, on peut citer l'amidon, le dextrane, l'agarose, la cellulose, la chitine, le chitosane, l'acide alginique, les glycosaminoglycanes, l'acide hyaluronique, et la chondroïtine sulfate ou leurs dérivés. L'amidon, le dextrane ou l'acide hyaluronique sont préférés, l'amidon étant tout particulièrement préféré.

Le polyaldéhyde peut être obtenu en ajoutant à la solution de polysaccharide ou mucopolysaccharide, une solution d'acide périodique ou l'un de ses sels jusqu'à l'obtention d'une concentration finale comprise entre 0,01 et 1 M, de préférence entre 0,25 et 0,5 M. L'étape d'oxydation peut être opérée sur des solutions, des gels ou des suspensions de polysaccharide(s).

La préparation de polysaccharide oxydé peut ensuite être soumise à des dialyses, diafiltrations, filtrations, ultrafiltrations, dans le but d'éliminer les produits de la réaction d'oxydation et des réactifs ainsi que des dérivés iodés formés pendant la réaction, ou en excès.

Avant utilisation, le polysaccharide ou mucopolysaccharide oxydé est conservé de préférence en solution acide, au pH qu'il acquiert spontanément, à une concentration comprise entre 0,5 et 20 % en poids, de préférence entre 1 et 10 %.

La solution est stable à l'abri de l'air et est conservée de préférence entre +1°C et +25°C.

Dans une variante, le polysaccharide ou mucopolysaccharide oxydé peut être sous forme lyophilisée acide, la redissolution du lyophilisat pouvant se faire en eau ou avec le tampon physiologique nécessaire.

Les polymères hydrophiles utiles aux fins de l'invention présentent de préférence un poids moléculaire de 1000 à 15000 Da, de préférence entre 2000 et 5000. Ils comprennent, par exemple, les dérivés de poly(éthylène) glycol (PEG), les poly(oxyéthylène), les poly(méthylène glycol), les poly(triméthylène glycol), les poly(vinylpyrrolidone), les dérivés du PEG étant les plus préférés. Ils peuvent être linéaires ou ramifiés, mais ne sont pas fortement réticulés. Les polymères en bloc poly(oxyéthylène)-pofy(oxypropylène) ayant éventuellement un noyau éthylène diamine (polymère à 4 fins de chaînes) peuvent également convenir.

Les polymères hydrophiles sont « activés » pour réagir sélectivement avec les amines et les thiols des protéines. En fin de chaînes des polymères, on trouve une structure semblable à : -chaîne du polymère-liant-GP (Groupe partant) pour les polymères réagissant avec les amines ou -chaînes du polymère-GRT (Groupe réactif vis-à-vis des thiols) pour les polymères réagissant avec les thiols.

Le liant peut être sélectionné parmi des groupes consistant en un carbonate -C(O)-, en un monoester -R-CH₂-C(O)- ou un diester -C(O)-O-(CH₂)ₙ-O-C(O)-, le GP peut être un dérivé succinimidyle, maléimidyle, phtalimidyle, imidazolyle, nitrophényle, trésyle ..., le dérivé succinimidyle étant le plus préféré. Enfin, les GTR peuvent être choisis parmi les dérivés vinylsulfone, iodoacétamide, maléimide et orthopyridyle-disulfure.

Ces polymères hydrophiles sont synthétisés suivant des méthodes connues de l'Homme de l'art.

Ils peuvent être conservés sous forme déshydratée, conditionnés en seringues.

Pour l'obtention de la matrice adhésive selon un premier mode de réalisation, les composés protéiques précités, notamment le collagène, le collagène chauffé ou l'albumine peuvent être en solution aqueuse. Ils sont mélangés extemporanément à l'agent de polymérisation/réticulation, dans des conditions telles que la polymérisation/réticulation desdits composés protéiques puisse se faire en un temps de préférence inférieur à 5 minutes.

Selon un deuxième mode de réalisation pour l'obtention de la matrice adhésive, les composés protéïques précités, notamment le collagène, le collagène chauffé ou l'albumine peuvent être sous forme solide, notamment de poudre sèche éventuellement stérilisée, par exemple dans une première seringue. Dans ce mode de réalisation, on préfère prévoir une étape supplémentaire pour solubiliser la poudre avant introduction de l'agent de polymérisation / réticulation. On peut alors utiliser une deuxième seringue contenant une solution aqueuse tamponnée. L'une au moins des deux seringues est associée à des moyens de chauffage pour permettre le réchauffement du mélange à une température de 37 à 50°C.

La mise en solution du composé protéïque est effectuée par des transferts successifs du contenu des deux seringues de l'une dans l'autre, en utilisant au mieux la possibilité de réchauffage qui facilite la solubilisation rapide du composé protéïque.

Lorsque le mélange est en suspension aqueuse homogène, il est alors possible d'introduire l'agent de réticulation pour continuer la préparation et l'application de la mousse protéïque adhésive comme dans le premier mode de réalisation.

Dans les deux modes de réalisation, que le composé protéïque soit en solution préformée ou en poudre sèche, il est préférable de partir de préparations stériles pour les applications chirurgicales.

Cette stérilité peut être obtenue à partir du moment où la matière première est stérilisée par filtration, en travaillant ensuite dans un environnement stérile (locaux stériles spéciaux, équipements préstérilisés et en atmosphère isolée).

Il est toutefois avantageux de pouvoir simplifier les conditions opératoires et d'en diminuer la complexité et le coût en adoptant un procédé validé de stérilisation finale. Une telle stérilisation peut être obtenue par irradiation gamma ou béta de préférence lorsque la solution ou la poudre protéique a été préalablement additionnée d'un agent piégeur de radicaux libres, "radioprotecteur" tel qu'un sucre ou un polysaccharide, notamment l'amidon à une concentration voisine de 1 %.

Le temps de polymérisation/réticulation peut être contrôlé selon les constituants mis en oeuvre pour l'obtention de la matrice adhésive, d'une manière connue en soi.

Selon un mode de réalisation de l'invention, la matrice adhésive est obtenue à partir du mélange d'un composé protéique en solution, de préférence du collagène natif, du collagène chauffé ou de l'albumine, avec un polysaccharide ou mucopolysaccharide oxydé, de préférence l'amidon oxydé, le dextrane oxydé, ou l'acide hyaluronique oxydé.

La matrice adhésive peut ainsi être préparée, selon un premier mode de réalisation de l'invention, à partir d'un mélange de polyaldéhyde et de collagène chauffé, dans un rapport en poids de 1 :10 à 1 :160, de préférence de 1 :15 à 1 :50, avec une concentration finale en collagène chauffé de 4 à 16%, de préférence de 4 à 13% en poids. La température de la solution de polysaccharide est de préférence comprise entre +1°C et +30°C et celle de la solution de collagène chauffé à une valeur permettant sa fluidification, soit entre +37°C et +50°C.

La température du mélange adhésif est comprise de préférence entre +35°C et +41 °C. Le temps de réaction du mélange peut être ajusté en fonction du pH du collagène chauffé, variant entre 6,5 et 7,5. Un temps court de polymérisation, inférieur à 1 minute, peut être obtenu à pH 7,5 et être progressivement augmenté en acidifiant la solution de collagène chauffé jusqu'à pH 6,5.

Selon un autre mode de réalisation de l'invention, la matrice adhésive est préparée à partir d'un mélange de polyaldéhyde oxydé et de collagène natif dans un rapport en poids de 1 :10 à 1 :50, de préférence de 1 :10 à 1 :30, avec une concentration finale en collagène de 1 à 5%, de préférence de 2 à 4%. La température de la solution de polysaccharide oxydé est de préférence comprise entre +1°C et +30°C et celle de la solution de collagène natif entre +18°C et +37°C. La température du mélange adhésif est comprise de préférence entre +18°C et +37°C. Le temps de réaction du mélange peut être ajusté en fonction du pH du collagène, entre 6,5 et 7,5 et de la température du mélange. Le temps de polymérisation augmente en diminuant le pH et/ou la température du mélange.

Selon un autre mode de réalisation de l'invention, la matrice adhésive est préparée à partir d'un mélange de polymère hydrophile activé en poudre et de collagène chauffé en solution, dans un rapport en poids de 1 :50 à 1 :1, de préférence entre 1 :10 à 1 :1, avec une concentration finale de collagène chauffé de 4 à 20 %, de préférence entre 10 et 18 %. La température de la solution de collagène chauffé est comprise entre +37 et +50°C et le pH de la solution de collagène chauffé peut varier de 6,9 à 9,0, suivant le temps de réticulation souhaité, de moins d'une minute à plusieurs dizaines de minutes. La température du mélange adhésif résultant est comprise de préférence entre +35 et +41°C.

Selon encore un autre mode de réalisation, on peut utiliser un mélange de polyaldéhyde oxydé et d'albumine de 1 :4.

Selon un mode de réalisation de l'invention, la matrice adhésive peut être préparée à partir de protéines ayant subi une coupure oxydative.

Dans ce cas, on peut mettre en oeuvre un traitement par l'acide périodique ou l'un de ses sels, de préférence le périodate de sodium, selon un procédé connu en soi.

Le collagène est particulièrement préféré aux fins de l'invention et peut être de tout type indiqué précédemment. Les préférences indiquées ci-dessus s'appliquent également dans ce cas.

La modification par coupure oxydative du collagène est décrite dans le brevet US 4 931 546.

Ce traitement provoque des coupures dans certains constituants du collagène, l'hydroxylysine et les sucres et crée ainsi des sites réactifs (groupes aldéhydes) sans en provoquer la réticulation tant que le pH de la solution de collagène reste acide.

Le collagène oxydé peut être conservé sous forme lyophilisée, à une température de +4°C à +25°C.

Selon ce mode de réalisation, l'agent de polymérisation/réticulation est alors formé d'un tampon à pH légèrement alcalin pour permettre la réticulation du mélange, à pH neutre.

Selon l'invention, la matrice adhésive peut ainsi être obtenue par mélange de collagène oxydé sous forme déshydratée avec un tampon en solution, la solution pouvant elle-même résulter de la dissolution préalable d'un tampon sous forme déshydratée dans l'eau.

Selon un autre mode de réalisation de l'invention, on peut utiliser pour la préparation de la matrice adhésive des protéines modifiées par un agent acylant ou sulfonant.

Les protéines indiquées précédemment ainsi que leurs préférences s'appliquent également à ce mode de réalisation.

L'agent de polymérisation/réticulation est également dans ce cas un tampon de pH légèrement alcalin à neutre, de préférence compris entre 6,0 et 9,0, plus préférentiellement entre 8,0 et 8,5.

La matrice adhésive est obtenue par un procédé similaire à celui indiqué précédemment en mélangeant les protéines à groupe acylant ou sulfonant avec la solution tampon pour que la réaction d'acylation ou de sulfonation puisse se produire en conduisant à la matrice adhésive.

Selon un autre mode de réalisation de l'invention où la matrice adhésive est à base de colle de fibrine, les colles actuellement disponibles sur le marché, notamment celles vendues sous les noms « Tissucol® » ou « Tisseel® » commercialisées par Baxter, « Beriplast® » commercialisée par Centéon, peuvent être utilisées aux fins de l'invention.

Il s'agit d'une solution concentrée de fibrinogène (70-140 mg/ml) contenant du facteur XIII et éventuellement de la fibronectine.

L'agent de polymérisation/réticulation consiste, dans ce cas, en une solution de thrombine (4-100 U.I) pouvant éventuellement être additionnée de collagène.

Conformément à l'invention, quel que soit le type de matrice adhésive choisi, la mousse adhésive est préparée lors de la formation de la matrice adhésive.

Lorsque celle-ci résulte du mélange de deux constituants de base (composé protéique - agent de polymérisation/réticulation), notamment dans les cas précités, ce mélange est réalisé extemporanément et avant son application sur les tissus. Lors de cette opération, un gaz est introduit par tout procédé connu de l'homme de l'art.

Le gaz peut être introduit notamment lors du mélange des constituants, ou directement dans le mélange préalablement formé (c'est-à-dire dans le matériau fluide de matrice protéique adhésive).

Le gaz utilisé aux fins de l'invention peut consister en de l'air ou en l'un ou plusieurs de ses composants, par exemple azote, oxygène, gaz carbonique.

Les gaz préférés sont l'air, le dioxyde de carbone et l'azote.

Il peut s'agir d'un gaz ou d'un mélange de gaz (désignés ci-après par le terme général « gaz »).

Conformément à l'invention, le gaz utilisé pour la formation de la mousse adhésive peut être associé, de manière préférée, à l'un des constituants de base pour la formation de la matrice adhésive, le cas échéant au composé protéique polymérisable/réticulable et/du à l'agent de polymérisation/réticulation, et/ou apporté indépendamment de l'un de ces constituants.

Le terme « associé » désigne le cas où le gaz est simplement contenu dans le même récipient que le constituant de la matrice adhésive (phases poudre/gaz ou liquide/gaz) comme le cas où le gaz est mélangé à l'agent de polymérisation/réticulation qui est par exemple sous forme pulvérulente ou lyophilisée.

Lorsque le gaz est associé à l'un des composants pour la matrice adhésive, la mousse est formée lors du mélange desdits composants pour l'obtention de la matrice adhésive.

Le gaz peut aussi être apporté indépendamment seul ou associé à un véhicule qui est non toxique, biocompatible et biodégradable et qui est mélangé avec la matrice adhésive et ses éléments constitutifs au moment de la préparation de la mousse adhésive.

Il peut s'agir d'un composé protéique tel que celui mis en oeuvre pour la formation de la matrice adhésive. Toutefois, dans ce cas, la quantité de composé protéique servant de véhicule est telle qu'il ne peut permettre la formation de la matrice adhésive à lui seul.

Le véhicule peut renforcer ou compléter l'activité de la mousse ou présenter une activité biologique. Il peut en particulier constituer parallèlement un véhicule pour une (des) substance(s) biologiquement active(s) comme indiqué ci-après.

Dans ce cas, le mélange pour la formation de la matrice adhésive peut être préalablement réalisé (pour conduire au matériau de matrice adhésive) puis le gaz éventuellement associé à un véhicule tel que décrit précédemment, est alors introduit dans la matrice adhésive déjà en cours de formation.

L'agent de polymérisation/réticulation et/ou le véhicule contenant le gaz se présente de préférence sous forme déshydratée, en particulier lyophilisée.

En variante, le véhicule peut être, de manière moins préférée, sous forme liquide.

Selon un autre aspect de l'invention, d'autres composants n'interférant pas avec la formation de la mousse peuvent être incorporés.

La mousse adhésive peut ainsi permettre la délivrance de substances biologiquement actives sur le site cible où elle est appliquée.

Une grande variété de substances biologiquement actives peut ainsi être mélangée au véhicule. Des exemples de telles substances incluent, mais de façon non limitative : drogues, vitamines, facteurs de croissance, hormones, dérivés stéroïdes, antibiotiques, vaccins, anti-viraux, antifongiques, anti-parasites, anti-tumoraux, anti-cancéreux, toxines, enzymes, inhibiteur d'enzymes, protéines, peptides, composés minéraux (ex. dérivés du zinc, du cuivre, du sélénium, du calcium) neurotransmetteurs, lipoprotéines, glycoprotéines, immuno-modulateurs, immunoglobulines et fragments de ceux-ci, agents de contraste, dérivés d'acides gras, polysaccharides, acides nucléiques (ex. fragments d'ADN, d'ARN) et polynucléotides.

Parmi les facteurs de croissance, les facteurs suivants ou leurs gènes correspondants sont particulièrement préférés : facteurs de type EGF (Endothelial Growth Factor), FGF (Fibroblast Growth Factor), TGF-β Transforming Growth Factor -β) incluant les BMP (Bone Morphogenetic Protein), IGF (Insulin Growth Factor), PDGF (Platelet Derived Growth Factor) VEGF (Vascular Endotheline Growth Factor) ou analogues et dérivés de ces facteurs.

Ces substances biologiquement actives peuvent être mélangées en solution avec le véhicule, puis éventuellement déshydratées par tout moyen connu de l'homme de l'art.

Il est possible également de reprendre un véhicule déshydraté dans un volume minimal de solution contenant la(les) substance(s) biologiquement active(s) ou d'ajouter une solution concentrée de cette(ces) substance(s) biologiquement active(s) à un véhicule déshydraté.

Enfin, de façon moins préférée, il est possible également de réaliser une solution aqueuse du véhicule mélangé à une(des) substance(s) biologiquement active(s) avant d'être mélangé au gaz.

Tout procédé connu de l'homme de l'art peut être employé pour réaliser la mousse, consistant simplement à mélanger différents produits et un gaz de manière homogène. Le mélange est réalisé extemporanément avant utilisation pour obtenir une mousse adhésive prête à l'emploi.

A cet effet, on peut utiliser les kits qui font l'objet de la présente invention.

Les constituants nécessaires à la formation de la mousse sont de préférence contenus, séparément, dans des seringues, la mousse étant obtenue par transfert en va-et-vient du contenu d'une seringue dans l'autre jusqu'à l'obtention d'un mélange homogène.

La mousse recueillie dans une seringue unique peut ensuite être appliquée au site désiré.

A cet effet, on peut utiliser un kit tel que celui décrit dans la demande WO 98/15299 pour la préparation d'une colle à base de collagène et de polyaldéhyde macromoléculaire.

On rappelle que ce kit peut se présenter sous la forme de deux seringues contenant respectivement le composant collagénique et le polyaldéhyde.

Les seringues sont fixées à un dispositif de maintien équipé de moyens de mélange conçus pour pouvoir mélanger extemporanément leur contenu de manière homogène, après avoir réchauffé la seringue de collagène à la température appropriée comprise entre 37°C et 50°C selon la fluidité recherchée.

Le composé protéique et l'agent de polymérisation/réticulation sont conditionnés sous une des formes décrites ci-dessus.

La quantité de gaz nécessaire est présente également dans l'une des seringues ou partagée dans chacune d'elles de sorte que le gaz est introduit au moment de la formation du matériau de matrice adhésive par mélange des constituants.

En variante, la quantité de gaz nécessaire éventuellement associée à un véhicule comme décrit ci-dessus, peut provenir d'une autre seringue, auquel cas il est introduit dans le matériau de matrice adhésive en cours de formation par exemple par polymérisation/réticulation, après mélange des constituants de base, ce pré-mélange formant le matériau de matrice adhésive pouvant lui-même être obtenu à l'aide du kit selon la demande WO 98/15299.

Lorsqu'il est mélangé à un véhicule, éventuellement combiné à une(des) substance(s) biologiquement active(s), le gaz représente de préférence au minimum 50 % du volume total de la préparation, et de façon plus préférée, 90 % du volume total.

Le mélange se fait de préférence en incorporant un volume de gaz représentant 25 à 90% du volume total de la mousse, de préférence de 40 à 75%.

Ce mélange se fait par ailleurs à une température favorisant l'incorporation du gaz dans la colle biologique. Cette température est de préférence physiologique, de façon plus préférée comprise entre 18°C et 41°C.

Le cas échéant, ce mélange se fait au tout début de la réticulation, de préférence lorsque la viscosité initiale du mélange est la plus faible.

Les mousses obtenues conformément à l'invention présentent des propriétés adhésives satisfaisantes pour une utilisation en chirurgie et/ou en thérapeutique, comparables à celles des colles biologiques connues à base desquelles elles sont réalisées.

Elles doivent être utilisées aussitôt, dans les cinq premières minutes de la préparation.

Selon les éléments constitutifs de la matrice adhésive et son mode d'obtention, on peut de manière connue contrôler le temps de polymérisation/réticulation pour permettre la formation de la mousse et son application au site désiré.

La mousse adhésive objet de la présente invention est appliquée immédiatement après sa formation lorsqu'elle est encore en cours de polymérisation/réticulation.

Elle est applicable par des procédés connus de l'homme de l'art. Elle est de préférence injectable, à travers des seringues, des cathéters, des canules ou tout autre matériel équivalent permettant un écoulement aisé de la mousse. Notamment, pour des dispositifs cylindriques, le diamètre intérieur peut être compris entre 0,1 à 2 mm. Le système d'injection peut comporter un applicateur dont la forme est particulièrement bien adaptée à l'utilisation souhaitée.

Selon une autre forme de réalisation de l'invention, la mousse peut être formée in situ par application subséquente des constituants nécessaires comme indiqué précédemment.

La mousse peut perdre son caractère collant après polymérisation/réticulation, en permettant une application sélective et précise sur les tissus cibles sans coller les tissus non désirés, voisins du site d'intervention.

La vitesse de prise en masse de la matrice adhésive dans la structure en mousse n'est pas affectée par l'introduction du gaz.

La mousse adhésive selon l'invention est non toxique et est parfaitement bien tolérée par l'organisme hôte, tout en étant plus élastique que les colles connues.

La densité de la mousse finale est variable selon la quantité de gaz introduite et l'application envisagée.

Elle est caractérisée par la présence de pores d'un diamètre compris généralement entre 50 et 200 microns.

Cette porosité confère au produit des propriétés remarquables vis-à-vis des plaquettes sanguines qui peuvent y adhérer plus rapidement grâce à la grande surface de contact extérieure. Il se forme un agrégat de plaquettes activées qui sécrètent les facteurs de coagulation nécessaires à l'hémostase. La matrice adhésive acquiert ainsi grâce à cette porosité des propriétés hémostatiques qui permettent d'arrêter des saignements par l'action conjuguée de l'étanchement mécanique de la plaie et de l'activation plaquettaire au contact du sang.

Cette porosité confère à la matrice adhésive une grande élasticité qui en fait un produit de choix pour étancher les plaies du poumon et stopper les fuites d'air tout en réalisant l'hémostase locale après exérèse d'une tumeur.

La porosité du matériau adhésif facilite sa colonisation cellulaire, sa biodégradation et sa transformation en tissu cicatriciel tout en évitant la formation d'adhérences postopératoires avec les organes adjacents à la plaie.

Les dépôts de mousse sur les tissus vivants sont notamment plus faciles à visualiser grâce à leur structure microporeuse particulière et leur opacité.

Grâce à sa faible densité, elle peut être appliquée avec une grande précision sur les tissus, sans connaître les problèmes de coulure habituellement rencontrés avec les colles liquides connues ou de dispersion par les gaz propulseurs des spray.

Sa fluidité initiale permet son injection à l'aide de seringues et son utilisation par laparoscopie à l'aide de canules et cathéters adaptés. Elle peut être étalée facilement à l'aide d'une spatule ou d'un pinceau par badigeonnage en chirurgie ouverte comme en laparoscopie.

Cet adhésif tissulaire poreux est donc particulièrement indiqué pour réaliser l'hémostase des plaies vasculaires ou tissulaires, chirurgicales ou traumatiques, les protéger et en faciliter la cicatrisation en évitant la formation d'adhérences postopératoires.

La mousse adhésive selon l'invention peut être mise en oeuvre, de façon non limitative, pour prévenir ou arrêter le saignement de plaies vasculaires ou tissulaires, pour lier des tissus biologiques, y compris des tissus vivants, entre eux ou à un biomatériau implanté, pour la cicatrisation de plaies chirurgicales ou chroniques, la protection ou l'étanchéité de sutures, la prévention de la formation d'adhérences post-opératoires, la délivrance de substances biologiquement actives notamment avec des médicaments pour une application locale, le comblement de cavités tissulaires (os, cartilage, lésions cutanées ...).

L'invention a donc également pour objet les procédés de traitements chirurgicaux ou médicaux comprenant la mise en place, en un site approprié de l'organisme, par une voie d'abord convenable, d'une quantité de mousse selon l'invention, efficace pour adhérer sur le site et provoquer l'effet recherché.

L'invention fournit ainsi un procédé de protection ou de liaison de tissus biologiques, y compris des tissus vivants, entre eux ou à un biomatériau implanté présentant des fonctions réactives vis-à-vis de l'un des constituants de la matrice adhésive, comprenant le fait de mélanger les constituants (constituants de la matrice adhésive et gaz) nécessaires à la formation de la mousse, simultanément ou successivement comme indiqué ci-dessus.

On applique ensuite rapidement la mousse fluide résultante, c'est-à-dire en moins de 3 minutes, pendant la polymérisation/réticulation de la matrice adhésive, sur lesdits tissus et/ou ledit biomatériau à une température comprise entre 20° et 41°C, puis on laisse polymériser/réticuler l'ensemble.

Le mélange préalable à l'application peut être réalisé avec le kit décrit précédemment.

Le temps de polymérisation/réticulation peut être ajusté en fonction des constituants de la matrice adhésive et de leur conservation, d'une manière connue en soi en faisant varier le pH, les concentrations, la température.

Le temps de résorption in vivo peut également être ajusté notamment en modifiant chimiquement les constituants de base de la matrice adhésive, comme cela est connu dans la technique ou en contrôlant la concentration en agent de polymérisation/réticulation.

Selon la composition de la matrice adhésive, ce temps peut varier de quelques jours à plusieurs mois.

Selon les applications, le biomatériau implanté consiste en la mousse adhésive elle-même qui est alors utilisée seule.

Dans d'autres cas, il peut s'agir de fixer un biomatériau présentant par exemple des fonctions aminées, réactives vis-à-vis du polyaldéhyde constitutif de la matrice adhésive.

Pour d'autres applications, notamment la prévention des adhérences post-opératoires, la mousse adhésive selon l'invention peut être utilisée seule ou être intimement liée à un film à base de collagène, pour former un matériau bicomposite.

Il peut s'agir d'un film collagénique tel que décrit dans WO 98/34656.

Le collagène mis en oeuvre pour former le film correspond à celui indiqué précédemment pour l'obtention de la mousse. Le collagène chauffé est préféré.

Le film collagénique peut comprendre en outre un additif hydrophile, de préférence chimiquement non réactif vis-à-vis du collagène, c'est-à-dire qui n'est pas susceptible de réagir avec le collagène présent, notamment qui ne forme pas de liaisons covalentes avec celui-ci lors de la réticulation.

L'additif hydrophile consiste de préférence en du polyéthylèneglycol.

La préparation du matériau bicomposite proprement dite est réalisée par l'assemblage de la couche formant film et de la mousse adhésive en cours de formation ou une fois formée, c'est-à-dire après mélange des constituants nécessaires.

L'assemblage comporte le coulage de la solution collagénique, destinée à réaliser le film, sur un support sensiblement plan adéquat, en répartissant celle-ci uniformément.

Le support est inerte en ce qu'il ne réagit pas avec les composants précités et n'intervient pas dans le processus de réticulation. Il est de préférence hydrophobe, par exemple en PVC ou polystyrène.

Cependant, ce support peut également être constitué d'un matériau pelliculable qui restera faiblement adhérent et qui pourra ensuite être séparé au moment de l'utilisation chirurgicale.

Ce support peut encore être lui-même constitué d'un film, par exemple de collagène séché, sur lequel on coule la solution, ou encore d'une couche de gel de matériau collagénique à un état de gélification nettement plus avancé.

La densité de la couche mince appliquée est de préférence comprise entre 0,1 et 0,3 g/cm².

Le coulage de cette solution collagénique est réalisé à une température avantageusement comprise entre 4 et 30°C, de préférence entre 18 et 25°C.

On laisse cette solution se gélifier et on applique sur ladite solution en cours de gélification, la mousse préparée comme indiqué précédemment. En d'autres termes, on dépose la couche de mousse poreuse sur le gel, l'application se poursuivant par simple gravité ou, éventuellement par une légère compression insuffisante pour provoquer un tassement sensible de la mousse.

Le moment auquel on applique la mousse poreuse sur la solution en cours de gélification est tel que le gel est encore mou et laisse pénétrer celle-ci sur une distance qui est avantageusement de l'ordre de 0,05 à 2 mm, de préférence de l'ordre de 0,1 à 0,5 mm.

En général, lorsque la solution qui se gélifie est à une température comprise entre 4 et 30°C, la couche de mousse poreuse est appliquée entre 5 et 30 minutes après la répartition de la solution sur la surface qui la reçoit.

On laisse sécher ou on lyophilise l'ensemble pour obtenir le matériau bicomposite selon l'invention.

La polymérisation/réticulation de la matrice adhésive peut s'effectuer ou s'achever, le cas échéant pendant le séchage du matériau bicomposite.

Ce séchage peut être obtenu à une température comprise entre 4 et 30°C, de préférence entre 18 et 25°C.

On peut réaliser le séchage du matériau dans un flux d'air stérile, si nécessaire.

Après séchage, le matériau bicomposite selon l'invention peut être séparé de son support. En variante, il peut comprendre ou incorporer un film ou une couche de matériau collagénique sur lequel la solution collagénique a été coulée.

Le matériau bicomposite selon l'invention est stable à température ambiante et reste stable pendant un temps suffisant pour sa manipulation à des températures pouvant aller jusqu'à 37-40°C.

L'épaisseur du film collagénique est de préférence inférieure à 100 µm, et plus préférentiellement comprise entre 30 et 75 µm.

L'épaisseur de la mousse est de préférence comprise entre 0,2 cm et 1,5 cm, plus préférentiellement encore entre 0,3 cm et 1,2 cm.

Un tel matériau bicouche présente un ensemble de qualités d'hémostase, d'anti-adhérences post-opératoires et de bio-dégradabilité particulièrement surprenantes.

Le matériau collagénique bicomposite selon l'invention est particulièrement adapté à la prévention d'adhérences post-opératoires, en particulier sur des plaies hémorragiques, du fait de la prévention des adhérences par le film, de la bonne adhésion du matériau composite sur de telles plaies et de l'absence de sang à l'interface.

Outre leurs propriétés d'hémostase et de prévention d'adhérences post-opératoires, le matériau collagénique relevant de la présente invention facilite la cicatrisation, à cause de sa structure composite, associant une couche très poreuse de mousse à un film collagénique.

La partie poreuse du matériau est facilement colonisable par les cellules environnantes. Le film protège la cicatrisation en cours pendant quelques jours grâce à ses propriétés d'étanchéité aux bactéries et microorganismes.

Le pouvoir de prévention des adhérences par le film du matériau est également renforcé par l'accélération de la cicatrisation de la plaie par la couche de mousse du matériau.

Selon l'invention, le matériau collagénique bicomposite est ainsi utile pour l'hémostase et la prévention des adhérences post-opératoires sur des plaies saignantes tout en facilitant la cicatrisation.

En outre, l'additif hydrophile macromoléculaire est éliminé par diffusion à travers le matériau collagénique, en quelques jours, matériau dont le gonflement favorise la dégradation du film collagénique en moins d'un mois.

Le matériau bicomposite selon l'invention peut également être utilisé pour favoriser la cicatrisation. Sa structure poreuse très ouverte permet une colonisation cellulaire rapide. Le film permet, quant à lui, d'isoler la partie poreuse pour la rendre accessible à des cellules spécifiques.

A titre d'exemple, des fibroblastes peuvent être cultivés dans la partie poreuse du matériau, in vitro, et des cellules épithéliales peuvent être cultivées sur le film en réalisant deux compartiments provisoirement séparés.

L'invention va être décrite plus en détails à l'aide des exemples donnés ci-après à titre indicatif et non limitatif.

### EXEMPLE 1 : Mousse adhésive constituée d'une matrice adhésive associant du collagène chauffé et de l'amidon oxydé (colle GAO)

### Préparation de l'amidon oxydé :

Une solution d'amidon soluble est préparée, à la concentration de 20 %, à la température de 75 °C, jusqu'à obtenir une solution parfaitement homogène, puis est dilué au ½. Elle est ensuite pré-filtrée et filtrée sur une membrane de porosité 0,22 µm.

Le pH de l'amidon est ajusté, alors, à pH 3,0-3,2 et la concentration de l'amidon à 6 %. On ajoute, ensuite, à la solution d'amidon oxydé du métaperiodate de sodium, à la concentration finale de 0,36 M, à température ambiante. Après 2 heures de traitement, la solution est dialysée avec une membrane de seuil de coupure allant de 5 à 10 kDa, contre de l'eau déminéralisée ultra-filtrée. La dialyse est poursuivie jusqu'à l'élimination totale des produits dialysables de la réaction d'oxydation et des réactifs ainsi que des dérivés iodés, formés pendant la réaction.

Ensuite, la solution d'amidon oxydé est ajustée, en concentration, à la valeur souhaitée, entre 1 à 3 %. Elle est pré-filtrée et filtrée stérilement sur membrane de porosité 0,22 µm.

Le produit est stable pendant au moins un an, à une température de +4 à +25 °C, à l'abri de l'air.

Pour la réalisation d'une mousse adhésive, la solution d'amidon oxydé peut être conditionnée en seringues.

La solution d'amidon oxydé, conditionnée en seringues ou en flacons, peut être également lyophilisée dans des conditions stériles et conservée à une température de +4 à +25°C, à l'abri de l'air.

La mise en solution ultérieure de l'amidon oxydé lyophilisé permet de préparer, si nécessaire, des solutions d'amidon oxydé plus concentrées pouvant atteindre 3 à 30 %.

### Préparation du collagène chauffé :

Le collagène utilisé est de source connue de l'homme de l'art. De type I bovin, il peut être acido-soluble ou solubilisé par digestion à la pepsine. D'origine de placenta humain, il peut être préparé par extraction à la pepsine, selon le procédé décrit dans le brevet EP-A-0 214 035.

On obtient par exemple un mélange des types I et III. Celui-ci peut être ensuite éventuellement utilisé pour séparer le type I et/ou le type III. Le collagène peut être aussi préparé par les techniques de recombination génétique.

On prépare une solution acide de collagène à une concentration de 4 à 16 % par addition progressive d'une poudre de collagène acide dans l'eau, à une température de 42 °C. Très rapidement, après 2 à 5 minutes d'agitation, dès que la fluidité le permet, la solution est neutralisée avec une solution molaire de soude, à un pH variant de 6,5 à 7,5.

Après neutralisation, la température de la solution de collagène est ajustée à +60 °C pour permettre sa stérilisation par filtration sur membrane de porosité 0,22 µm, à la suite de pré-filtrations.

Pour une utilisation en kit notamment comme décrit dans la demande WO 98/15299, le collagène est ensuite réparti dans des seringues de manière stérile et est conservé à une température comprise entre +4 et +25 °C, tout en étant stable pendant au moins un an.

Dans une variante, la solution de collagène chauffé est additionnée d'amidon à 1 % ou d'autres agents protecteurs vis à vis des radiations ionisantes. L'ensemble est filtré à une température de 42°C sur membrane de porosité 0,22 microns et réparti en seringues qui peuvent être stérilisées par irradiation gamma, en final à une dose de 5 à 30 kilogreys

### Réalisation de la mousse adhésive GAO

On prend une seringue chauffante de 5 ml, remplie avec 2 ml de collagène chauffé à la concentration de 16 % et enveloppée par un film résistif équipé d'un thermostat permettant de maintenir la température du collagène entre +44 et +50°C. On prépare également une seringue de 5 ml contenant 2,5 ml d'air et 0,5ml d'amidon oxydé.

On mélange, ensuite, le contenu de ces deux seringues raccordées par un simple connecteur, en chassant alternativement totalement le contenu de l'une dans l'autre, 10 à 20 fois jusqu'à obtenir une mousse adhésive complètement homogène.

Selon une autre variante de réalisation de la mousse adhésive, on prend une seringue chauffante de 2,5 ml, remplie avec 2 ml de collagène chauffé à la concentration de 16 % et enveloppée par un film résistif équipé d'un thermostat permettant de maintenir la température du collagène entre +44 et +50°C. On prépare d'un autre côté une seringue contenant 0,5 ml d'amidon oxydé. Ces deux seringues sont assemblées en un kit tel que décrit dans la demande de brevet WO 98/15299. Elles sont réunies par un ensemble connecteur / mélangeur dont la fonction est d'obtenir un gel adhésif parfaitement homogène. Le contenu du kit est transféré dans une seringue vide de 5 ml, à l'aide d'un simple raccord. En parallèle, on prépare une seringue de 5 ml contenant 2,5 ml d'air.

On mélange, ensuite, le contenu de ces deux seringues comme décrit plus haut.

Selon une autre variante il est possible de préparer une mousse adhésive de densité moitié de la précédente. Pour cela, on assemble un kit pour 'colle' biologique, en utilisant une seringue de 2,5 ml remplie avec 2 ml de collagène chauffé à la concentration de 16 % et une seringue contenant 0,5 ml d'amidon oxydé. Le contenu de ce kit est déversé dans une seringue de 10 ml. On prépare d'un autre côté une seringue de 10 ml renfermant 7,5 ml d'air.

On mélange, ensuite, le contenu des deux seringues suivant le procédé décrit ci-dessus.

### EXEMPLE 2 : Mousse constituée d'une matrice adhésive préparée à partir de « colle GAO » et de collagène natif.

### Préparation du collagène natif

On prépare une solution de collagène à 3 % dans de l'eau ultrafiltrée déminéralisée. On ajoute ensuite une solution de phosphate disodique 0,22 M, pour obtenir une concentration finale de 20 mM. On homogénéise la suspension de collagène avec un agitateur à pale défloculeuse, puis on ajuste son pH à 7,4-7,5, avec une solution concentrée d'acide chlorhydrique.

La suspension de collagène neutralisé est, ensuite, diluée avec de l'eau ultrafiltrée déminéralisée pour atteindre une concentration en collagène de 1,8 % et en phosphate de 13 mM. Elle est laissée au repos, pendant une nuit, pour avoir une fibrillation complète du collagène.

Le lendemain, la suspension de collagène est centrifugée à 10000-15000 G, pour concentrer le précipité de collagène, qui est homogénéisé, par suite, avec un agitateur à pale défloculeuse. Deux grammes de précipité de collagène à 2 %, en poids, sont répartis dans des seringues de 5 ml qui sont lyophilisées, dans des conditions connues de l'homme de l'art.

Après la lyophilisation, on introduit le piston des seringues de collagène, sans comprimer le collagène. On conditionne ces seringues dans un double emballage étanche qui sont stérilisées par gamma-irradiation à une dose de 25 à 35 Kgy.

Deux autres variantes principales sont applicables pour la préparation de cette poudre de collagène natif stérile.
a) la suspension de collagène précipité à 2 % est additionnée de 1 % d'amidon avant lyophilisation, ce qui permet de diminuer les effets hydrolytiques de l'irradiation finale stérilisante sur la molécule de collagène.
b) la suspension de collagène est préparée de manière stérile tout au long du procédé pour éviter l'irradiation finale stérilisante.

D'autres variantes de ce procédé sont d'introduire des quantités et des concentrations plus ou moins importantes de collagène.

### Préparation des éléments de la « colle GAO »

Les éléments de la colle GAO sont préparés comme décrit dans l'exemple 1 et comprennent une seringue de collagène chauffé à la concentration de 8 % et une seringue d'amidon oxydé à la concentration de 1,5 %.

### Réalisation de la mousse adhésive GAO / collagène natif

Comme décrit dans l'exemple précédent, on réalise d'abord le mélange du collagène chauffé et de l'amidon oxydé (colle GAO), aux concentrations respectives de 8 et 1,5 %. Pour cela, on peut utiliser un kit tel que décrit dans la demande WO 98/15299 et transférer 2,5 ml de gel, dans une seringue de 5 ml. On peut également utiliser deux seringues de 5 ml raccordées par un simple connecteur, l'une contenant 2 ml de collagène chauffé à 8 % et l'autre 0,5 ml d'amidon oxydé à 1,5 %. Le mélange des deux produits est réalisé en chassant alternativement totalement le contenu de l'une des deux seringues dans l'autre, 5 à 10 fois jusqu'à obtenir un gel complètement homogène.

Le collagène utilisé pour cet exemple est du collagène bovin de type I, extrait de derme de veau, éventuellement solubilisé par digestion à la pepsine et, purifié par des précipitations salines, selon les techniques déjà décrites. On peut utiliser, de la même manière, des collagènes d'autres espèces animales ou d'origine humaine de type I, de type III, ou d'origine recombinante, ou d'autres types ou leur mélange en toutes proportions.

La seringue de collagène lyophilisé est ensuite raccordée avec la seringue de colle GAO mélangée. Les deux produits sont homogénéisés, en commençant par faire passer la colle GAO dans la seringue contenant le collagène lyophilisé, puis en transférant le contenu d'une seringue à l'autre, en poussant tour à tour leurs pistons, 10 à 20 fois, jusqu'à obtenir une mousse homogène.

A partir du moment où l'on prépare la colle GAO, la mousse doit être réalisée et utilisée avant que la matrice adhésive soit entièrement polymérisée pour qu'elle puisse adhérer aux tissus.

### EXEMPLE 3 : Mousse constituée d'une matrice adhésive préparée à partir de colle GAO et de collagène natif mélangé au FGF (Fibroblast Growth Factor)

La colle GAO est préparée comme décrit dans l'exemple précédent, à partir de 2 ml de collagène chauffé à 8 % et 0,5 ml d'amidon oxydé à 1,5 %. Elle est transférée dans une seringue de 5 ml.

A la seringue de collagène natif, on ajoute 100 à 250 µl d'une solution de FGF humain recombinant.

Puis, on mélange cette seringue de collagène natif à la colle GAO, comme décrit précédemment jusqu'à obtenir une mousse homogène GAO/collagène natif - FGF.

Selon une autre variante, on attend un temps d'adsorption du FGF sur le collagène natif, de 5 à 120 minutes, avant de procéder au mélange de la préparation de collagène / FGF à la colle GAO.

Une autre variante de cet exemple consiste à lyophiliser le FGF avec le collagène natif suivant le procédé ci-dessous. On mélange au précipité de collagène une solution de FGF qui est homogénéisé, réparti dans des seringues de 5 ml, à raison de 2 g par seringue, lyophilisé et soit préparé stérilement soit stérilisé par gamma-irradiation. Cette seringue de collagène et FGF lyophilisé est mélangé à 2,5 ml de colle GAO, comme décrit dans l'exemple 2, jusqu'à obtenir une mousse homogène.

La composition de cette mousse adhésive est particulièrement employée pour le comblement de lésions nerveuses, le FGF étant un facteur facilitant la régénération des nerfs.

Dans cet exemple, on peut remplacer le FGF par d'autres facteurs de croissance ou leurs mélanges, possédant des activités équivalentes au FGF.

### EXEMPLE 4 : Mousse constituée d'une matrice adhésive préparée à partir de colle GAO et de collagène natif mélangé à l'IL-2 (interleukine de type 2)

On reprend l'exemple 3 en remplaçant le FGF ou facteurs équivalents par l'IL-2.

Cette mousse adhésive GAO/collagène natif - IL-2 est particulièrement intéressante dans le contrôle de la cancérogenèse et l'inhibition du développement de tumeurs. Elle peut être également préparée avec d'autres produits, seuls ou mélangés qui inhibent le développement de cancers et de tumeurs.

### EXEMPLE 5 : Mousse constituée d'une matrice adhésive préparée à partir de colle GAO et de collagène natif mélangé à des facteurs de croissance cellulaire ou de régénération tissulaire

Les exemples 3 et 4 peuvent être répétés avec du collagène natif mélangé à tout facteur de croissance cellulaire ou de régénération tissulaire pour préparer des mousses adhésives actives sur des plaies cutanées, osseuses, cartilagineuses, ...

### EXEMPLE 6 : Mousse adhésive préparée à partir de collagène en poudre sèche.

### Préparation du collagène :

On prépare une solution acide de collagène à une concentration de 2 % par addition progressive d'une poudre de collagène acide dans l'eau, à une température de 20-25 °C. Dès que la solution est parfaitement homogène, le collagène est neutralisé par ajout de phosphate de sodium, à la concentration finale de 10 mM, pour atteindre un pH de 6,5-8. La solution de collagène est ensuite laissée au repos pendant 1 nuit, à 20-25°C, puis le collagène précipité est récupéré par centrifugation. Il est dessalé et déshydraté par une série de lavages acétoniques : dans l'ordre, 1 bain acétone/eau, 90/10, m/m, 3 bains acétone/eau, 80/20, m/m et 3 bains acétone 100 %.

Le collagène est ensuite réparti dans un volume de 2,5 ml dans des seringues de 5 ml, à raison de 80-400 mg de collagène sec par seringue.

Le collagène utilisé est de source connue de l'homme de l'art incluant les collagènes recombinants. De type I bovin, il peut être acido-soluble ou solubilisé par digestion à la pepsine. D'origine de placenta humain, il peut être préparé par extraction à la pepsine, selon le procédé décrit dans le brevet EP-A-0,214,035.

Le collagène après une filtration stérilisante initiale peut être préparé stérilement tout au long du procédé dans des locaux stériles et avec des équipements appropriés connus de l'homme de l'art.

Dans une variante, le collagène réparti dans une seringue Becton-Dickinson Réf : "STERIFILL" peut être stérilisé par gamma-irradiation à la dose de 5 à 35 kilogrey, de préférence en présence d'un agent protecteur vis à vis des effets hydrolytiques des irradiations, tel que l'amidon. Un volume d'air additionnel est incorporé dans la seringue si nécessaire pour augmenter le volume futur de la mousse.

### Préparation d'une seringue d'eau distillée ou de tampon physiologique stérile, selon les méthodes habituelles, en utilisant une même seringue Becton-Dickinson.

Cette seringue peut contenir aussi un volume d'air complémentaire. D'une manière avantageuse l'une des deux seringues est équipée ou associée avec un système de chauffage permettant une régulation de la température de 30 à 50°C.

### Préparation de la mousse :

Après chauffage de l'une des deux seringues, celle-ci est connectée avec l'autre seringue à l'aide d'un connecteur de diamètre intérieur voisin de 2 mm, assez large pour éviter le colmatage par des particules et grumeaux initiaux de collagène.

Le contenu de la seringue liquide est envoyé dans la seringue contenant la poudre et le mélange est réalisé par transferts successifs, 10 à 20 fois, à une température inférieure à 37°C lorsque l'on veut conserver la structure hélicoïdale du collagène et de 37 à 50°C lorsque l'on veut obtenir une diminution ou une suppression de la structure hélicoïdale.

Lorsque la mousse est homogénéisée, elle est conservée dans l'une des deux seringues (à température tiède ou ambiante suivant la seringue utilisée) avant d'être mélangée avec une seringue stérile contenant l'amidon oxydé, à température ambiante, préparée comme dans les exemples précédents.

Après incorporation de l'amidon oxydé dans la mousse précédente, la mousse finale est à une température inférieure à 40°C, le plus souvent voisine de 37°C, et doit être utilisée dans les cinq minutes suivantes, tant qu'elle est encore suffisamment fluide.

La vitesse de réticulation est facilement contrôlable par l'ajustement du pH du collagène utilisé et de sa concentration.

Dans des variantes, la seringue de poudre de collagène ou de la solution aqueuse de reprise peuvent être additionnées de produit(s) biologique(s) apportant des fonctions biologiques complémentaires telles que antibiotiques, anti-inflammatoires, facteurs de croissance etc...

### EXEMPLE 7 : Mousse adhésive constituée d'une matrice adhésive associant l'albumine et l'amidon oxydé (colle AAO)

On prépare comme décrit dans l'exemple 1 une solution d'amidon oxydé de 10 à 25 %.

### Préparation de l'albumine

L'albumine utilisée est de source connue. D'origine humaine ou animale ou issue des techniques de recombinaison génétique.

L'albumine est reprise à une concentration de 20 à 50 %, neutralisée à pH 6,5-7,5 avec des solutions concentrées de soude et d'acide chlorhydrique et filtrée stérilement sur membrane de porosité 0,22 µm. 2 ml de cette solution sont ensuite conditionnés dans des seringues de 5 ml.

La seringue d'albumine et une seringue contenant 0,5 ml d'amidon oxydé de 10 à 25 % sont assemblées en un kit, suivant le procédé décrit dans l'exemple 1. Le kit est appelé AAO.

Réalisation de la mousse adhésive AAO / collagène natif.

Comme décrit dans les exemple précédents, on peut d'abord réaliser le mélange de l'albumine et de l'amidon oxydé, aux concentrations respectives de 20-50 et de 10 à 25 %, sans introduire d'air. Pour cela, on peut utiliser un kit semblable à celui utilisé pour la préparation de la colle GAO de l'exemple 2 et transférer 2,5 ml de gel, dans une seringue de 5 ml.

La seringue de colle AAO est ensuite raccordée à une seringue de 5 ml contenant 2,5 ml d'air. Les deux produits sont homogénéisés, en commençant par faire passer l'air dans la seringue contenant la colle AAO, puis en transférant le contenu d'une seringue à l'autre, en poussant tour à tour leurs pistons, 10 à 20 fois, jusqu'à obtenir une mousse homogène d'un volume de 5 ml.

On peut également utiliser deux seringues de 5 ml raccordées par un simple connecteur, l'une contenant 2 ml d'albumine à 20-50 % et l'autre 0,5 ml d'amidon oxydé de 10 à 25 % et 2,5 ml d'air. Le mélange des deux produits est réalisé en chassant alternativement totalement le contenu de l'une des deux seringues dans l'autre, 5 à 10 fois jusqu'à obtenir une mousse complètement homogène.

A partir du moment où l'on prépare la colle AAO, la mousse doit être réalisée et utilisée avant que la matrice adhésive soit entièrement polymérisée pour qu'elle puisse adhérer aux tissus.

### EXEMPLE 8 : Mousse adhésive préparée à partir d'albumine déshydratée et d'amidon oxydé en solution.

- Préparation de l'albumine
   0,4 à 1,25 g d'albumine en poudre sont conditionnés de manière stérile dans une seringue Becton-Dickinson de 5 ml réf : "STERIFILL"
- Préparation d'une seringue de 2 ml d'eau distillée ou de solution physiologique PBS associée à un système de chauffage qui permet de porter la température du liquide entre 37 et 45°C.
   Les deux seringues sont ensuite connectées bout à bout à l'aide d'un raccord de 1 à 2 mm de diamètre.
   On mélange ensuite le contenu des deux seringues par transferts successifs de l'une dans l'autre. Après 10 à 20 transferts, la mousse homogène d'albumine est recueillie dans l'une des deux seringues.
   Celle-ci est ensuite connectée à une seringue contenant 0,5 ml d'amidon oxydé à 6 % et après un mélange par transferts successifs d'une seringue dans l'autre, on peut appliquer la mousse protéïque adhésive sur la plaie à traiter.

### EXEMPLE 9 : Mousse adhésive constituée d'une matrice adhésive associant l'albumine et des dérivés de polyéthylènes glycols portant des groupes électrophiles activés réactifs vis-à-vis des amines.

L'albumine est reprise à une concentration de 20 à 50 %, neutralisée à pH 6,5-9 comme décrit dans l'exemple 7 ou 8. 2 ml de cette solution sont ensuite conditionnés dans des seringues de 5 ml.

Parmi les PEG électrophiles activés, on peut utiliser indifféremment, seul ou mélangés en toutes proportions, le SPA-PEG (succinimidyl propionate PEG), le SCM-PEG (Succinimidyl ester of carboxymethylated PEG) et le BTC-PEG (Benzotriazole carbonate of PEG) de poids moléculaire supérieur à 1000 Da, dérivés de PEG, produits commercialisés par Shearwater Polymers. On peut également utiliser le PEG-SS2 (Disuccinimidyl succinate PEG), synthétisé comme décrit dans la demande de brevet WO 96/03159 (Minnesota Mining and Manufacturing Company). On conditionne 40 à 500 mg de PEG activé sous forme déshydratée par seringue de 5 ml.

### Réalisation de la mousse adhésive.

On réalise le mélange de l'albumine et des PEG activés en transférant la totalité du contenu de la seringue d'albumine dans celle du PEG, puis en chassant entièrement le contenu de l'une des deux seringues dans l'autre, 5 à 10 fois jusqu'à obtenir un gel complètement homogène de 5 ml.

A partir du moment où l'on prépare la colle associant l'albumine et les dérivés électrophiles de PEG activé, la mousse doit être réalisée et utilisée avant que la colle soit entièrement polymérisée pour qu'elle puisse adhérer aux tissus.
La vitesse de polymérisation et donc le temps disponible pour utiliser le produit est réglé par le pH du mélange. Plus le pH est acide, plus la réaction est lente et plus long est le temps disponible.

### EXEMPLE 10 : Mousse adhésive constituée d'une matrice adhésive associant l'albumine et des dérivés de PEG activés réactifs vis-à-vis des sulfhydryles.

L'albumine est reprise à une concentration de 20 à 50 %, neutralisée à pH 6,5-9 comme décrit dans les exemples précédents. 2 ml de cette solution sont ensuite conditionnés dans des seringues de 5 ml.

Parmi les PEG activés réactifs vis-à-vis des sulfhydryles, on peut utiliser indifféremment, seul ou mélangés, en toutes proportions, le VS-PEG (vinyl sulfone PEG), le MAL-PEG (Maléimide PEG) et le OPSS-PEG (orthopyridyl-disulfide PEG) de poids moléculaire supérieur à 1000 Da, dérivés de PEG, produits commercialisés par Shearwater Polymers. On conditionne 40 à 500 mg de PEG activé et déshydraté par seringue de 2 ml.

La mousse adhésive est ensuite réalisée comme décrit dans l'exemple 9.

### EXEMPLE 11 : Mousse adhésive constituée d'une matrice adhésive associant le collagène chauffé et des dérivés de polyéthylènes glycols portant des groupes électrophiles activés réactifs vis-à-vis des amines.

On reprend l'exemple 9 en remplaçant l'albumine par le collagène chauffé, préparé comme décrit dans l'exemple 1, à la concentration de 10-20 %, à pH 6,5-9 et en conditionnant deux fois moins de PEG activé et déshydraté par seringue de 5 ml, soit 20-250 mg de PEG.

### EXEMPLE 12 : Mousse adhésive constituée d'une matrice adhésive associant le collagène chauffé et des dérivés PEG activés réactifs vis-à-vis des sulfhydryles.

On reprend l'exemple 10 en remplaçant l'albumine par le collagène chauffé, préparé comme décrit dans l'exemple 1, à la concentration de 10-20 %, à pH 6,5-9 et en conditionnant deux fois moins de PEG activé et déshydraté par seringue de 5 ml, soit 20-250 mg de PEG.

### EXEMPLE 13 : Mousse adhésive constituée d'une matrice adhésive préparée à partir d'une colle de fibrine et de gel d'agarose.

### Préparation du gel d'agarose (véhicule)

On reprend en solution de l'agarose, à la concentration finale de 0,5-5 %, dans de l'eau déminéralisée apyrogène, à une température comprise entre 75 et 100 °C, puis on ajuste le pH de cette solution à pH 7,5-9 avec un tampon phosphate concentré pour obtenir une concentration finale en phosphate de 10-20 mM. On transfère 2 ml de cette solution, par seringue de 5 ml. Cette solution est ensuite lyophilisée, dans des conditions connues de l'homme de l'art.

Dans une autre variante, la solution d'agarose est réalisée dans de l'eau déminéralisée apyrogène, tamponnée avec 10-20 mM de borax à un pH 7,5-9 ou avec un mélange - 1:1, mol/mol - de borax et de phosphate, à une concentration finale de 10-20 mM.

Après la lyophilisation, on introduit le piston dans les seringues, sans comprimer l'agarose. On conditionne ces seringues dans un double emballage étanche qui sont stérilisées par gamma-irradiation à une dose de 25 à 35 KGy.

Une autre variante de ce procédé est de filtrer stérilement la solution d'agarose, à chaud ∼ c'est-à-dire dès que la viscosité de la solution est suffisamment basse pour permettre la filtration stérilisante de la solution -, après l'ajustement de son pH à 7,5-9 comme indiqué ci-dessus, sur membranes de porosité 0,22 à 0,45 µm. Cette solution est ensuite répartie stérilement dans des seringues de 5 ml, à raison de 2 ml par seringue, et lyophilisée. Après la lyophilisation, on introduit le piston dans les seringues, sans comprimer l'agarose. On conditionne ces seringues dans un double emballage étanche. Toutes les opérations effectuées, après la filtration stérilisante, sont réalisées dans des conditions stériles connues de l'homme de l'art.

### Réalisation de la mousse adhésive colle de fibrine / agarose

On réalise d'abord extemporanément la colle de fibrine. Toutes les colles de fibrine du commerce peuvent convenir. Il peut s'agir par exemple d'une solution de TISSUCOL® contenant le fibrinogène. 2 ml de solution de colle de fibrine préparée extemporanément sont transférés dans une seringue de 5 ml et chauffés à 40°C.

La seringue d'agarose lyophilisé est ensuite raccordée avec la seringue de colle de fibrine. Les deux produits sont homogénéisés, en commençant par faire passer la colle de fibrine dans la seringue contenant l'agarose lyophilisé, puis en transférant le contenu d'une seringue à l'autre, en poussant tour à tour leurs pistons, 10 à 20 fois, jusqu'à obtenir une mousse homogène.

A partir du moment où l'on prépare la colle de fibrine, la mousse doit être réalisée et utilisée avant que le fibrinogène soit entièrement transformé en fibrine.

### EXEMPLE 14 : Mousse adhésive constituée d'une matrice adhésive préparée à partir de colle de fibrine, d'agarose et d'un antibiotique.

On reprend l'exemple 11 en ajoutant à 2 ml de solution d'agarose avant qu'elle soit lyophilisée, 0,25-2,5 mg de vancomycine, un antibiotique efficace contre les bactéries gram-positives, notamment Staphylococcus aureus et Staphylococcus epidermidis, les deux agents principaux d'infection de greffes dans la chirurgie vasculaire.

Cette formulation est utilisée pour l'étanchéité de points de sutures d'anastomoses vasculaires.

Une variante de cet exemple est d'inclure à la place de la vancomycine d'autres antibiotiques, seuls ou leurs mélanges en toutes proportions.

## Revendications

1. Procédé pour l'obtention d'une mousse protéique adhésive fluide, biocompatible, biorésorbable et non toxique, à usage chirurgical et/ou thérapeutique, notamment pour la protection/cicatrisation de plaies tissulaires et pour la liaison de tissus biologiques entre eux ou à un biomatériau implanté, **caractérisé en ce qu'**il comprend le fait de mélanger extemporanément, de manière homogène :
- un composé protéique polymérisable/réticulable et potentiellement adhésif contenu dans une première seringue,
à
- un agent de polymérisation/réticulation contenu dans une deuxième seringue,
pour former un matériau fluide de matrice protéique adhésive biocompatible, biorésorbable et non toxique, et
- un gaz ou un mélange de gaz biocompatibles et non toxiques choisi parmi l'air, l'azote, l'oxygène ou le gaz carbonique ou le mélange d'un ou plusieurs de ces gaz, contenu dans la première et/ou la deuxième seringue et/ou une troisième seringue, avec ce matériau fluide de matrice protéique adhésive, ou avec un des constituants de base d'un tel matériau solubilisé en milieu aqueux,
**par transfert en va-et-vient du mélange entre deux seringues.**

2. Procédé selon la revendication 1, **caractérisé en ce que** le composé protéique sous forme solide, notamment sous forme de fibres ou de poudre sèche, est mélangé extemporanément avec une solution aqueuse tamponnée à l'aide de moyens de chauffage et **en ce que** l'on amène dans le mélange l'agent de polymérisation/réticulation.

3. Procédé selon l'une quelconque des revendications 1 et 2, **caractérisé en ce que** le composé protéique est constitué de, ou comprend, une protéine ou un mélange de protéines choisies parmi le collagène, la gélatine, l'albumine, l'élastine et le fibrinogène, de préférence parmi le collagène et l'albumine.

4. Procédé selon l'une quelconque des revendications 1 à 3, **caractérisé en ce que** le composé protéique est constitué de, ou comprend, du collagène.

5. Procédé selon la revendication 4, **caractérisé en ce que** le composé protéique est constitué de, ou comprend, du collagène natif, du collagène natif chimiquement modifié, notamment par méthylation, par succinylation, par coupure oxydative notamment à l'aide d'acide périodique ou l'un de ses sels, du collagène natif sans télopeptides, du collagène ayant perdu au moins partiellement sa structure hélicoïdale, constitué majoritairement de chaînes α et dont le poids moléculaire est voisin de 100 kDa, non hydrolysé (collagène chauffé).

6. Procédé selon la revendication 4, **caractérisé en ce que** le composé protéique est constitué de, ou comprend, du collagène chauffé.

7. Procédé selon la revendication 1, **caractérisé en ce que** le composé protéique consiste en, ou comprend, du collagène natif sous forme de solution aqueuse à une concentration comprise entre 1 et 5 %, de préférence 2,5 et 4 % en poids.

8. Procédé selon la revendication 1, **caractérisé en ce que** le composé protéique consiste en, ou comprend, du collagène chauffé solubilisé en milieu aqueux à une concentration comprise entre 4 et 20 %, de préférence entre 5 et 18 % en poids.

9. Procédé selon l'une quelconque des revendications 1 et 3, **caractérisé en ce que** le composé protéique consiste en, ou comprend, de l'albumine, solubilisée en milieu aqueux à une concentration comprise entre 20 et 50 %, de préférence 40 à 50%.

10. Procédé selon l'une quelconque des revendications 1 à 9, **caractérisé en ce que** l'agent de polymérisation/réticulation est un polymère réactif de poids moléculaire supérieur à 1000, de préférence choisi parmi les polyaldéhydes macromoléculaires et les polymères hydrophiles capables de réagir avec le composé protéique, notamment vis-à-vis de fonctions amines ou sulfhydryles.

11. Procédé selon la revendication 10, **caractérisé en ce que** le polyaldéhyde macromoléculaire est choisi parmi les polysaccharides ou mucopolysaccharides oxydés, de préférence parmi l'amidon, le dextrane, l'agarose, la cellulose, la chitine, le chitosane, l'acide alginique, les glycosaminoglycanes, l'acide hyaluronique et la chondroïtine sulfate, et leurs dérivés ou leurs mélanges, plus préférentiellement parmi l'amidon, le dextrane et l'acide hyaluronique.

12. Procédé selon la revendication 11, **caractérisé en ce que** le polyaldéhyde macromoléculaire comprend l'amidon oxydé.

13. Procédé selon la revendication 10, **caractérisé en ce que** le polymère hydrophile est choisi parmi les dérivés de poly (éthylène) glycol (PEG), les poly (oxyéthylène), les poly (méthylène glycol), les poly (triméthylène glycol), les poly (vinylpyrrolidone), les dérivés du PEG étant les plus préférés.

14. Procédé selon la revendication 10, **caractérisé en ce que** l'agent de polymérisation/réticulation est un polyaldéhyde macromoléculaire solubilisé en milieu aqueux à une concentration comprise entre 0,5 et 10% en poids, de préférence entre 1 et 3% en poids.

15. Procédé selon l'une quelconque des revendications 1 à 8 et 10 à 14, **caractérisé en ce que** le composé protéique consiste en, ou comprend, du collagène natif ou du collagène chauffé et l'agent de polymérisation/réticulation est l'amidon oxydé.

16. Procédé selon l'une quelconque des revendications 1 à 3, 8 et 14, 15, **caractérisé en ce que** la proportion de polyaldéhyde macromoléculaire au collagène chauffé est de 1/10 à 1/160, de préférence de 1/15 à 1/50, la température de mélange étant comprise entre 35° C et 41 ° C.

17. Procédé selon l'une quelconque des revendications 1 à 4 et 10 à 15, **caractérisé en ce que** la proportion de polyaldéhyde macromoléculaire au collagène natif est comprise entre 1/10 à 1/50, de préférence 1/10 à 1/30 et la température de mélange est comprise entre18° C et 37° C.

18. Procédé selon l'une quelconque des revendications 1 à 3, **caractérisé en ce que** le composé protéique a été préalablement modifié chimiquement ou par coupure oxydative, notamment par traitement à l'acide périodique ou l'un de ses sels, et **en ce que** l'agent de polymérisation est formé d'un tampon à pH légerement alcalin pour permettre la poiymérisation/réticulation du composé protéique à un pH sensiblement neutre.

19. Procédé selon l'une quelconque des revendications 1 à 3, **caractérisé en ce qu'**il comprend le fait de mélanger en solution aqueuse du fibrinogène avec de la thrombine.

20. Procédé selon l'une quelconque des revendications 1 à 19, **caractérisé en ce que** le gaz est choisi parmi l'air, l'azote, l'oxygène et le gaz carbonique ou le mélange d'un ou plusieurs de ces gaz, de préférence parmi l'air, le dioxyde de carbone et l'azote, l'air étant tout particulièrement préféré.

21. Procédé selon l'une quelconque des revendications 1 à 20, **caractérisé en ce que** le gaz est associé à l'un ou plusieurs des constituants pour la matrice protéique adhésive.

22. Procédé selon l'une quelconque des revendications 1 à 21, **caractérisé en ce que** le gaz est associé à un véhicule, biocompatible et non toxique, de préférence formé d'un composé protéique selon la revendication 3.

23. Procédé selon l'une quelconque des revendications 1 à 21, **caractérisé en ce que** le gaz est fourni à l'aide de l'agent de polymérisation/réticulation et/ou du véhicule sous forme pulvérulente ou lyophilisée.

24. Procédé selon l'une quelconque des revendications 1 à 21, **caractérisé en ce que** le gaz est fourni à l'aide du composé protéique sous forme pulvérulente ou lyophilisée.

25. Procédé selon l'une quelconque des revendications 1 à 24, **caractérisé en ce que** le volume de gaz introduit représente 25 à 90 % du volume total de la mousse adhésive, de préférence 40 à 75 %.

26. Procédé selon l'une quelconque des revendications 1 à 25, **caractérisé en ce qu'**il comprend le fait d'introduire dans le matériau de matrice protéique adhésive une ou plusieurs substances biologiquement actives.

27. Procédé selon la revendication 26, **caractérisé en ce que** la ou les substances biologiquement active(s) est (sont) associée(s) à un véhicule, biocompatible et non toxique, qui est éventuellement le véhicule pour le gaz ou le mélange de gaz.

28. Procédé selon l'une quelconque des revendications 1 à **27, caractérisé en ce que** le gaz est introduit dans le matériau de matrice adhésive (matrice en cours de formation).

29. Procédé selon l'une quelconque des revendications 1 à **27, caractérisé en ce que** le gaz est introduit au moment du mélange des constituants pour la formation de la matrice adhésive.

30. Procédé selon l'une quelconque des revendications 1 à **29, caractérisé en ce que** le gaz est mélangé avec le matériau de matrice adhésive pour donner une température comprise entre18° C et 41° C.

31. Kit pour la préparation d'une mousse protéique adhésive fluide biocompatible, biorésorbable et non toxique, à usage chirurgical et/ou thérapeutique, notamment pour la protection/cicatrisation de plaies tissulaires et la liaison de tissus biologiques entre eux ou un biomatériau implanté, **caractérisé en ce qu'**il comprend :
- un composé protéique polymérisable/réticulable, potentiellement adhésif, solubilisé en milieu aqueux, dans une première seringue,
- un agent de polymérisation/réticulation dans une deuxième seringue,
pour former une matrice protéique adhésive fluide biocompatible, biorésorbable et non toxique, et
- un gaz ou un mélange de gaz biocompatibles et non toxiques, choisi parmi l'air, l'azote, l'oxygène ou le gaz carbonique ou le mélange d'un ou plusieurs de ces gaz dans la première, deuxième et/ou dans une troisième seringue,
et des moyens pour mélanger extemporanément les constituants, composé protéique en solution aqueuse et agent de polymérisation/réticulation, pour former la matrice adhésive et ledit gaz ou mélange de gaz.

32. Kit selon la revendication **31**, **caractérisé en ce qu'**il comporte un premier récipient contenant le composé protéique potentiellement adhésif sous forme pulvérulente, déshydratée et éventuellement stérilisée, un second récipient contenant une solution aqueuse tamponnée éventuellement stérile, des moyens pour amener un agent de polymérisation/réticulation dans le composé protéique solubilisé et des moyens pour mélanger le contenu des premier et second récipients, et des moyens pour mettre en oeuvre un gaz dans ledit mélange et obtenir la mousse.

33. Kit selon l'une quelconque des revendications **31 et 32, caractérisé en ce que** le composé protéique, l'agent de polymérisation/réticulation et le gaz sont tels que définis selon l'une quelconque des revendications 3 à 27.

34. Kit selon la revendication **31, caractérisé en ce qu'**il se présente sous la forme de deux seringues équipées de moyens de mélange dont l'une des seringues contient le composé protéique en solution aqueuse et l'autre contient l'agent de polymérisation/réticulation.

35. Kit selon l'une quelconque des revendications **31 à 34, caractérisé en ce que** le gaz est associé au composé protéique et/ou à l'agent de polymérisation/réticulation.

36. Kit selon la revendication **32, caractérisé en ce que** lesdits moyens de mélange permettent de faire passer plusieurs fois le mélange d'une seringue à l'autre pour assurer la formation de la mousse à partir du gaz compris dans la seringue contenant le composé protéique pulvérulent.

37. Kit selon l'une quelconque des revendications **31 à 35, caractérisé en ce que** le gaz est associé à un véhicule biocompatible et non toxique, de préférence formé d'un composé protéique selon la revendication 3.

38. Kit selon l'une quelconque des revendications **31 à 35, caractérisé en ce qu'**il comprend une troisième seringue contenant le gaz éventuellement associé à un véhicule.

39. Kit selon la revendication **38, caractérisé en ce que** le véhicule contient en outre une ou plusieurs substances biologiquement actives.

40. Kit selon l'une quelconque des revendications **31 à 39, caractérisé en ce que** l'agent de polymérisation/réticulation et/ou le véhicule est sous forme lyophilisée.

## Patentansprüche

1. Verfahren zum Erhalt eines bioverträglichen, bioresorbierbaren und nicht toxischen Eiweißschaum-Klebstofffluids zur chirurgischen oder therapeutischen Verwendung, insbesondere für den Schutz/die Vernarbung von Gewebewunden und für das Verbinden von biologischen Geweben untereinander oder an ein implantiertes Biomaterial, **dadurch gekennzeichnet, daß** es die Stufe des Mischens an Ort und Stelle in homogener Weise umfaßt von:
- einer polymerisierbaren/vernetzbaren und potentiell klebenden Eiweißverbindung, die in einer esten Spritze enthalten ist,
- eines Mittels zur Polymerisation/Vernetzung, das in einer zweiten Spritze enthalten ist, zur Bildung eines fluiden Materials von klebender, bioverträglicher, bioresorbierbarer und nicht toxischer Eiweißmatrix, und
- einem Gas oder einer Mischung von Gasen, das/die bioverträglich und nicht toxisch ist, ausgewählt unter Luft, Stickstoff, Sauerstoff oder Kohlenstoff enthaltenden Gasen oder der Mischung von einem oder mehreren dieser Gase, welches/welche in der ersten und/oder der zweiten Spritze und/oder einer dritten Spritze enthalten ist, mit diesem fluiden Material von Eiweiß-Klebstoffmatrix oder mit einem der Grundbestandteile eines solchen Materials, das in wässrigem Milieu solubilisiert ist,
mittels Überführung durch Hin-und-Herbewegen des Gemisches zwischen zwei Spritzen.

2. Verfahren nach Anspruch 1, **dadurch gekennzeichnet, daß** die Eiweißverbindung in fester Form, insbesondere in Form von trockenen Fasern oder trockenem Pulver, an Ort und Stelle mit einer gepufferten wässrigen Lösung mit Hilfe von Erwärmungsmitteln gemischt wird, und daß man in das Gemisch ein Mittel zur Polymerisation/Vernetzung einführt.

3. Verfahren nach irgendeinem der Ansprüche 1 und 2, **dadurch gekennzeichnet, daß** die Eiweißverbindung aus einem Protein oder einer Mischung von Proteinen, ausgewählt unter Kollagen, Gelatine, Albumin, Elastin und Fibrinogen, bevorzugt unter Kollagen und Albumin, gebildet wird oder dieses umfaßt.

4. Verfahren nach irgendeinem der Ansprüche 1 bis 3, **dadurch gekennzeichnet, daß** die Eiweißverbindung durch Kollagen gebildet wird oder dieses umfaßt.

5. Verfahren nach Anspruch 4, **dadurch gekennzeichnet, daß** die Eiweißverbindung aus natürlichem Kollagen, natürlichem Kollagen, das chemisch modifiziert ist insbesondere durch Methylierung, Succinylierung, durch oxidatives Schneiden mittels Perjodsäure oder einem ihrer Salze, natürlichem Kollagen ohne Telopeptide, nicht hydrolysiertem Kollagen (erhitztes Kollagen), das wenigstens partiell seine Spiralstruktur verloren hat, gebildet hauptsächlich durch α-Ketten, und dessen Molekulargewicht in der Nähe von 100 kDa liegt, gebildet wird diese oder umfaßt.

6. Verfahren nach Anspruch 4, **dadurch gekennzeichnet, daß** die Eiweißverbindung durch erhitztes Kollagen gebildet wird oder dieses umfaßt.

7. Verfahren nach Anspruch 1, **dadurch gekennzeichnet, daß** die Eiweißverbindung durch natürliches Kollagen in Form von wässriger Lösung mit einer Konzentration zwischen 1 und 5 Gew.-%, bevorzugt 2,5 und 4 Gew.-%, gebildet wird oder dieses umfaßt.

8. Verfahren nach Anspruch 1, **dadurch gekennzeichnet, daß** die Eiweißverbindung durch erhitztes, in wässrigem Milieu solubilisiertes Kollagen mit einer Konzentration zwischen 4 und 20 Gew.-%, bevorzugt 5 und 18 Gew.-%, gebildet wird oder dieses umfaßt.

9. Verfahren nach irgendeinem der Ansprüche 1 und 3 **dadurch gekennzeichnet, daß** die Eiweißverbindung durch in wässrigem Milieu solubilisiertes Albumin mit einer Konzentration zwischen 20 und 50 %, bevorzugt 40 bis 50 %, gebildet wird oder dieses umfaßt.

10. Verfahren nach irgendeinem der Ansprüche 1 bis 9, **dadurch gekennzeichnet, daß** das Mittel zur Polymerisation/-Vernetzung ein reaktionsfähiges Polymeres mit einem Molekulargewicht oberhalb 1000 ist, bevorzugt ausgewählt unter makromolekularen Polyaldehyden und hydrophilen Polymeren, die zur Reaktion mit der Eiweißverbindung fähig sind, insbesondere gegenüber Amin- oder Hydrogensulfidfunktionen.

11. Verfahren nach Anspruch 10, **dadurch gekennzeichnet, daß** der makromolekulare Polyaldehyd ausgewählt ist unter den oxidierten Polysacchariden oder Mucopolysacchariden, insbesondere unter Stärke, Dextran, Agarose, Cellulose, Chitin, Chitosan, Alginsäure, den Glucoaminoglycanen, Hyaluronsäure und Chondroitinsulfat, und deren Derivaten oder deren Mischungen, mehr bevorzugt unter Stärke, Dextran und Hyaluronsäure.

12. Verfahren nach Anspruch 11, **dadurch gekennzeichnet, daß** der makromolekulare Polyaldehyd oxidierte Stärke umfaßt.

13. Verfahren nach Anspruch 10, **dadurch gekennzeichnet, daß** das hydrophile Polymere ausgewählt ist unter den Derivaten von Poly(ethylen)glykol (PEG), den Poly(oxyethylenen), den Poly(methylenglykolen), den Poly(trimethylenglykolen), den Poly(vinylpyrolidonen), wobei die Derivate von PEG mehr bevorzugt sind.

14. Verfahren nach Anspruch 10, **dadurch gekennzeichnet, daß** das Mittel zur Polymerisation/Vernetzung ein in wässrigem Milieu solubilisierter makromolekularer Polyaldehyd mit einer Konzentration zwischen 0,5 und 10 Gew.-%, bevorzugt zwischen 1 und 3 Gew.-%, ist.

15. Verfahren nach irgendeinem der Ansprüche 1 bis 8 und 10 bis 14, **dadurch gekennzeichnet, daß** die Eiweißverbindung aus natürlichem Kollagen oder erhitztem Kollagen besteht oder dieses umfaßt, und das Mittel zur Polymerisation/Vernetzung oxidierte Stärke ist.

16. Verfahren nach irgendeinem der Ansprüche 1 bis 3, 8 und 14, 15, **dadurch gekennzeichnet, daß** das Verhältnis von makro-molekularem Polyaldehyd zu erhitztem Kollagen von 1/10 bis 1/160, bevorzugt von 1/15 bis 1/50, beträgt, wobei die Tempe-ratur des Gemisches zwischen 35°C und 41°C liegt.

17. Verfahren nach irgendeinem der Ansprüche 1 bis 4 und 10 bis 15, **dadurch gekennzeichnet, daß** das Verhältnis von makromolekularem Polyaldehyd zu natürlichem Kollagen zwischen 1/10 bis 1/50, bevorzugt zwischen 1/10 bis 1/30, liegt, und wobei die Temperatur des Gemisches zwischen 18°C und 37°C liegt.

18. Verfahren nach irgendeinem der Ansprüche 1 bis 3, **dadurch gekennzeichnet, daß** die Eiweißverbindung vorher chemisch oder durch oxidatives Schneiden, insbesondere durch Behandlung mit Perjodsäure oder einem ihrer Salze, modifiziert wurde, und daß das Mittel zur Polymerisation durch einen Puffer mit leicht alkalischem pH gebildet wurde, um die Polymerisation/Vernetzung der Eiweißverbindung

19. Verfahren nach irgendeinem der Ansprüche 1 bis 3, **dadurch gekennzeichnet, daß** es die Stufe umfaßt des Mischens in wässriger Lösung des Fibrinogens mit Thrombin.

20. Verfahren nach irgendeinem der Ansprüche 1 bis 19, **dadurch gekennzeichnet, daß** das Gas ausgewählt ist unter Luft, Stickstoff, Sauerstoff und Kohlenstoff enthaltenden Gasen oder dem Gemisch von einem oder mehreren dieser Gase, bevorzugt Luft, Kohlendioxid und Stickstoff, wobei Luft besonders bevorzugt ist.

21. Verfahren nach irgendeinem der Ansprüche 1 bis 20, **dadurch gekennzeichnet, daß** das Gas mit einem oder mehreren Bestandteilen für die klebenden Eiweißmatrix assoziiert ist.

22. Verfahren nach irgendeinem der Ansprüche 1 bis 21, **dadurch gekennzeichnet, daß** das Gas mit einem bioverträglichen und nicht toxischen Träger, bevorzugt gebildet durch eine Eiweißverbindung gemäß Anspruch 3, assoziiert ist.

23. Verfahren nach irgendeinem der Ansprüche 1 bis 21, **dadurch gekennzeichnet, daß** das Gas mit Hilfe des Mittels zur Polymerisation/Vernetzung und/oder des Trägers in pulverförmiger oder lyophilisierter Form angeliefert wird.

24. Verfahren nach irgendeinem der Ansprüche 1 bis 21, **dadurch gekennzeichnet, daß** das Gas mit Hilfe der Eiweißverbindung in pulverförmiger oder lyophilisierter Form angeliefert wird.

25. Verfahren nach irgendeinem der Ansprüche 1 bis 24, **dadurch gekennzeichnet, daß** das Volumen des eingeführten Gases 25 bis 90 % des Gesamtvolumens des Klebstoffschaumes, bevorzugt 40 bis 75 %, ausmacht.

26. Verfahren nach irgendeinem der Ansprüche 1 bis 25, **dadurch gekennzeichnet, daß** es die Stufe der Einführung von einer oder mehreren biologisch aktiven Substanzen in das Material von Klebstoff-Eiweißmatrix umfaßt.

27. Verfahren nach Anspruch 26, **dadurch gekennzeichnet, daß** die biologisch aktive/n Substanz/en an einen bioverträglichen und nicht toxischen Träger assoziiert ist/sind, der gegebenenfalls der Träger für das Gas oder das Gasgemisch ist.

28. Verfahren nach irgendeinem der Ansprüche 1 bis 27, **dadurch gekennzeichnet, daß** das Gas in das Material der Klebstoffmatrix (Matrix im Verlauf der Bildung) eingeführt wird.

29. Verfahren nach irgendeinem der Ansprüche 1 bis 27, **dadurch gekennzeichnet, daß** das Gas im Augenblick des Mischens der Bestandteile zur Bildung der Klebstoffmatrix eingeführt wird.

30. Verfahren nach irgendeinem der Ansprüche 1 bis 29, **dadurch gekennzeichnet, daß** das Gas mit dem Material der Klebstoffmatrix zum Erhalt einer Temperatur zwischen 10° und 41°C gemischt wird.

31. Satz für die Herstellung eines bioverträglichen, bioresorbierbaren und nicht toxischen Eiweißschaum-Klebstofffluids zur chirurgischen oder therapeutischen Verwendung, insbesondere für den Schutz/die Vernarbung von Gewebewunden und für das Verbinden von biologischen Geweben untereinander oder an implantiertes Biomaterial, **dadurch gekennzeichnet, daß** er umfaßt:
- eine polymerisierbare/vernetzbare und potentiell klebende Eiweißverbindung, die in einer esten Spritze enthalten ist,
- ein Mittel zur Polymerisation/Vernetzung, das in einer zweiten Spritze enthalten ist, zur Bildung eines fluiden, bioverträglichen, bioresorbierbaren und nicht toxischen Eiweißklebstoffes, und
- ein Gas oder eine Mischung von Gasen, das/die bioverträglich und nicht toxisch ist, ausgewählt unter Luft, Stickstoff, Sauerstoff oder Kohlenstoff enthaltenden Gasen oder der Mischung von einem oder mehreren dieser Gase, in der ersten, zweiten und/oder einer dritten Spritze, und
- Mittel, um die Bestandteile, Eiweißverbindung in wässriger Lösung und Mittel zur Polymerisation/Vernetzung, zur Bildung der Klebstoffmatrix und dieses Gas oder dieses Gasgemisch an Ort und Stelle zu mischen.

32. Satz nach Anspruch 31, **dadurch gekennzeichnet, daß** er umfaßt: einen ersten Behälter, der die dehydratisierte und gegebenenfalls sterilisierte, potentiell klebende Eiweißverbindung in Pulverform enthält, einen zweiten Behälter, der eine wässrige, gepufferte, gegebenenfalls sterile Lösung enthält, Mittel zur Zuführung eines Mittels zur Polymerisation/-Vernetzung in die solubilisierte Eiweißverbindung und Mittel zum Mischen des Inhaltes der ersten und zweiten Behälter und Mittel zur Einführung eines Gases in dieses Gemisches und zum Erhalt des Schaumes.

33. Satz nach irgendeinem der Ansprüche 31 und 32, **dadurch gekennzeichnet, daß** die Eiweißverbindung, das Mittel zur Polymerisation/Vernetzung und das Gas solche sind, wie in irgendeinem der Ansprüche 3 bis 27 definiert.

34. Satz nach Anspruch 31, **dadurch gekennzeichnet, daß** er in Form von zwei Spritzen vorliegt, die mit Mitteln zum Mischen ausgerüstet sind, wovon eine der Spritzen die Eiweißverbindung in wässriger Lösung enthält, und die andere das Mittel zur Polymerisation/Vernetzung enthält.

35. Satz nach irgendeinem der Ansprüche 31 bis 34, **dadurch gekennzeichnet, daß** das Gas an der Eiweißverbindung und/oder an dem Mittel zur Polymerisation/Vernetzung assoziiert ist.

36. Satz nach Anspruch 32, **dadurch gekennzeichnet, daß** diese Mittel zum Mischen es erlauben, das Gemisch von einer Spritze zur anderen mehrmals zu überführen, um die Bildung des Schaumes mit Hilfe des Gases, das in der die pulverförmige Eiweißverbindung enthaltenden Spritze vorhanden ist, sicherzustellen.

37. Satz nach irgendeinem der Ansprüche 31 bis 35, **dadurch gekennzeichnet, daß** das Gas mit einem bioverträglichen und nicht toxischen Träger, bevorzugt gebildet von einer Eiweißverbindung gemäß Anspruch 3, assoziiert ist.

38. Satz nach irgendeinem der Ansprüche 31 bis 35, **dadurch gekennzeichnet, daß** er eine dritte Spritze umfaßt, welche das Gas, gegebenenfalls assoziiert mit einem Träger, enthält.

39. Satz nach Anspruch 38, **dadurch gekennzeichnet, daß** der Träger außerdem eine oder mehrere biologisch aktive Substanzen enthält.

40. Satz nach irgendeinem der Ansprüche 31 bis 39, **dadurch gekennzeichnet, daß** das Mittel zur Polymerisation/Vernetzung und/oder der Träger in lyophilisierter Form vorliegen.

## Claims

1. Method for obtaining a fluid adhesive protein foam which is biocompatible, bioresorbable and non-toxic, for surgical and/or therapeutic use, especially for protecting/cicatrising tissue wounds and for attaching biological tissues to each other or to an implanted biomaterial, **characterised in that** it includes the fact of mixing extemporaneously, in a homogeneous manner:
- a potentially adhesive protein compound which can be polymerised/cross-linked, contained in a first syringe,
with
- a polymerisation/cross-linking agent contained in a second syringe for forming a fluid adhesive protein matrix material which is biocompatible, bioresorbable and non-toxic, and
- a gas or a mixture of biocompatible and non-toxic gases selected from air, nitrogen, oxygen or carbon dioxide or the mixture of one or more of these gases, contained in the first and/or the second syringe and/or a third syringe, with this fluid adhesive protein matrix material, or with one of the basic constituents of such a material solubilised in aqueous medium,
**by reciprocating transfer of the mixture between two syringes.**

2. Method according to claim 1, **characterised in that** the protein compound in solid form, especially in the form of fibres or dry powder, is mixed extemporaneously with an aqueous solution buffered with the aid of heating means and **in that** the polymerisation/cross-linking agent is fed into the mixture.

3. Method according to any one of claims 1 and 2, **characterised in that** the protein compound consists in, or includes, a protein or a mixture of proteins selected from collagen, gelatin, albumin, elastin and fibrinogen, preferably from collagen and albumin.

4. Method according to any one of claims 1 to 3, **characterised in that** the protein compound consists in, or includes, collagen.

5. Method according to claim 4, **characterised in that** the protein compound consists in, or includes, native collagen, native collagen which has been chemically modified especially by methylation, by succinylation, by oxidative cutting especially with the aid of periodic acid or one of its salts, native collagen without telopeptides, collagen which has at last partially lost its helical structure, made up mainly of α chains and of which the molecular weight is close to 100 kDa, non-hydrolysed (heated collagen).

6. Method according to claim 4, **characterised in that** the protein compound consists in, or includes, heated collagen.

7. Method according to claim 1, **characterised in that** the protein compound consists in, or includes, native collagen in the form of an aqueous solution in a concentration of between 1 and 5 %, preferably between 2.5 and 4 % by weight.

8. Method according to claim 1, **characterised in that** the protein compound consists in, or includes, heated collagen solubilised in aqueous medium in a concentration of between 4 and 20 %, preferably between 5 and 18 % by weight.

9. Method according to any one of claims 1 and 3, **characterised in that** the protein compound consists in, or includes, albumin, solubilised in aqueous medium in a concentration of between 20 and 50 %, preferably between 40 and 50 %.

10. Method according to any one of claims 1 to 9, **characterised in that** the polymerisation/cross-linking agent is a reactive polymer having a molecular weight greater than 1000, preferably selected from the macromolecular polyaldehydes and hydrophilic polymers capable of reacting with the protein compound, especially with regard to amine or sulphhydryl functions.

11. Method according to claim 10, **characterised in that** the macromolecular polyaldehyde is selected from oxidised polysaccharides or mucopolysaccharides, preferably from starch, dextran, agarose, cellulose, chitin, chitosan, alginic acid, glycosamino-glycanes, hyaluronic acid and chondroitin sulphate, and their derivatives or mixtures, by particular preference from starch, dextran and hyaluronic acid.

12. Method according to claim 11, **characterised in that** the macromolecular polyaldehyde comprises oxidised starch.

13. Method according to claim 10, **characterised in that** the hydrophilic polymer is selected from the derivatives of poly(ethylene) glycol (PEG), poly (oxyethylene), poly(methylene glycol), poly (trimethylene glycol), poly (vinyl pyrrolidon), the derivatives of PEG being the most preferred.

14. Method according to claim 10, **characterised in that** the polymerisation/cross-linking agent is a macromolecular polyaldehyde solubilised in aqueous medium in a concentration of between 0.5 and 10 % by weight, preferably between 1 and 3 % by weight.

15. Method according to any one of claims 1 to 8 and 10 to 14, **characterised in that** the protein compound consists in, or includes, native collagen or heated collagen and the polymerisation/cross-linking agent is oxidised starch.

16. Method according to any one of claims 1 to 3, 8 and 14, 15, **characterised in that** the proportion of macromolecular polyaldehyde to heated collagen is from 1/10 to 1/160, preferably from 1/15 to 1/50, the mixing temperature being between 35°C and 41°C.

17. Method according to any one of claims 1 to 4 and 10 to 15, **characterised in that** the proportion of macromolecular polyaldehyde to native collagen is between 1/10 and 1/50, preferably between 1/10 and 1/30 and the mixing temperature is between 18°C and 37°C.

18. Method according to any one of claims 1 to 3, **characterised in that** the protein compound has been previously modified, chemically or by oxidative cutting, especially by treatment with periodic acid or one of its salts, and **in that** the polymerisation agent is formed from a buffer with a slightly alkaline pH in order to permit the polymerisation/cross-linking of the protein compound at a substantially neutral pH.

19. Method according to any one of claims 1 to 3, **characterised in that** it includes the fact of mixing fibrinogen with thrombin in aqueous solution.

20. Method according to any one of claims 1 to 19, **characterised in that** the gas is selected from air, nitrogen, oxygen and carbon dioxide or the mixture of one or more of these gases, preferably from air, carbon dioxide and nitrogen, air being very particularly preferred.

21. Method according to any one of claims 1 to 20, **characterised in that** The gas is associated with one or more of the constituents for the adhesive protein matrix.

22. Method according co any one of claims 1 to 21, **characterised in that** the gas is associated with a biocompatible and non-toxic vehicle, preferably formed from a protein compound according to claim 3.

23. Method according to any one of claims 1 to 21, **characterised in that** the gas is supplied with the aid of the polymerisation/cross-linking agent and/or the vehicle in pulverulent or lyophilised form.

24. Method according to any one of claims 1 to 21, **characterised in that** the gas is supplied with the aid of the protein compound in pulverulent or lyophilised form.

25. Method according to any one of claims 1 to 24, **characterised in that** the volume of gas introduced represents 25 to 90 % of the total volume of the adhesive foam, preferably 40 to 75 %.

26. Method according to any one of claims 1 to 25, **characterised in that** it includes the fact of introducing one or more biologically active substances into the adhesive protein matrix material.

27. Method according to claim 26, **characterised in that** the biologically active substance or substances is/are associated with a biocompatible and non-toxic vehicle, which is possibly the vehicle for the gas or the gas mixture.

28. Method according to any one of claims 1 to 27, **characterised in that** the gas is introduced into the adhesive matrix material (matrix in the process of forming).

29. Method according to any one of claims 1 to 27, **characterised in that** the gas is introduced at the moment of mixing the constituents for forming the adhesive matrix.

30. Method according to any one of claims 1 to 29, **characterised in that** the gas is mixed with the adhesive matrix material to give a temperature of between 12°C and 41°C.

31. Kit for the preparation of a fluid adhesive protein foam which is biocompatible, bioresorbable and non-toxic, for surgical and/or therapeutic use, especially for protecting/cicatrising tissue wounds and for attaching biological tissues to each other or to an implanted biomaterial, **characterised in that** it includes:
- a potentially adhesive protein compound which can be polymerised/cross-linked, solubilised in aqueous medium, in a first syringe,
- a polymerisation/cross-linking agent in a second syringe, for forming a fluid adhesive protein matrix which is biocompatible, bioresorbable and non-toxic, and
a gas or a mixture of biocompatible and non-toxic gases, selected from air, nitrogen, oxygen or carbon dioxide or the mixture of one or more of these gases in the first, second and/or a third syringe,
and means for mixing extemporaneously the constituents, protein compound in aqueous solution and polymerisation/cross-linking agent, for forming the adhesive matrix and the said gas or gas mixture.

32. Kit according to claim 31, **characterised in that** it includes a first receptacle containing the potentially adhesive protein compound in pulverulent form, dehydrated and possibly sterilised, a second receptacle containing a possibly sterile buffered aqueous solution, means for feeding a polymerisation/cross-linking agent into the solubilised protein compound and means for mixing the contents of the first and second receptacles, and means for using a gas in said mixture and obtaining the foam.

33. Kit according to any one of claims 31 and 32, **characterised in that** the protein compound, the polymerisation/cross-linking agent and the gas are as defined according to any one of claims 3 to 27.

34. Kit according to claim 31, **characterised in that** it is in the form of two syringes equipped with mixing means, one of which syringes contains the protein compound in aqueous solution and the other contains the polymerisation/cross-linking agent.

35. Kit according to any one of claims 31 to 34, **characterised in that** the gas is associated with the protein compound and/or with the polymerisation/cross-linking agent.

36. Kit according to claim 32, **characterised in that** said mixing means make it possible to pass the mixture a number of times from one syringe to the other to ensure the formation of the foam from the gas included in the syringe containing the pulverulent protein compound.

37. Kit according to any one of claims 31 to 35, **characterised in that** the gas is associated with a biocompatible and non-toxic vehicle, preferably formed from a protein compound according to claim 3.

38. Kit according co any one of claims 31 to 35, **characterised in that** it comprises a third syringe containing the gas possibly associated with a vehicle.

39. Kit according to claim 38, **characterised in that** the vehicle contains in addition one or more biologically active substances.

40. Kit according to any one of claims 31 to 39, **characterised in that** the polymerisation/cross-linking agent and/or the vehicle are/is in lyophilised form.
